# FASCICULE DE BREVET EUROPEEN

(11) **EP 0 996 731 B1**
(45) Date de publication et mention de la délivrance du brevet: **31.08.2005**
(21) Numéro de dépôt: 98936467.4
(22) Date de dépôt: 07.07.1998
(51) Int. Cl.: C12N 15/48, C12Q 1/70, C07K 14/15, A61K 31/70

(54) **MATERIEL NUCLEIQUE RETROVIRAL ET FRAGMENTS NUCLEOTIDIQUES NOTAMMENT ASSOCIES A LA SCLEROSE EN PLAQUES ET/OU LA POLYARTHRITE RHUMATOIDE, A DES FINS DE DIAGNOSTIC, PROPHYLACTIQUES ET THERAPEUTIQUES**
MULTIPLE SKLEROSE UND/ODER RHEUMATISCHE POLYARTHRITIS ASSOZIIERTE RETROVIRALE NUCLEINSÄUREN UND ENTSPRECHENDE NUCLEOTIDFRAGMENTE FÜR DIE DIAGNOSTIK, PROPHYLAXE UND THERAPIE
RETROVIRAL NUCLEIC MATERIAL AND NUCLEOTIDE FRAGMENTS, IN PARTICULAR ASSOCIATED WITH MULTIPLE SCLEROSIS AND/OR RHEUMATOID ARTHRITIS, FOR DIAGNOSTIC, PROPHYLACTIC AND THERAPEUTIC USES

(30) Priorité: 07.07.1997 FR 9708816
(43) Date de publication de la demande: 03.05.2000
(73) Titulaire: BIO MERIEUX, 69280 Marcy l'Etoile (FR)
(72) Inventeur: PARAHNOS-BACCALA, Glaucia, F-69003 Lyon (FR); KOMURIAN-PRADEL, Florence, F-69250 Poleymieux au Mont d'Or (FR); BEDIN, Frédéric, F-69002 Lyon (FR); SODOYER, Mireille, F-69110 Sainte Foy les Lyon (FR); OTT, Catherine, F-69007 Lyon (FR); MALLET, François, F-69100 Villeurbanne (FR); PERRON, Hervé, F-69005 Lyon (FR); MANDRAND, Bernard, F-69100 Villeurbanne (FR)
(74) Mandataire: Guerre, Dominique
(86) Numéro de dépôt international: PCT/FR1998/001460
(87) Numéro de publication internationale: WO 1999/002666

(56) Documents cités:
- WO-A-93/07259
- WO-A-94/28138
- WO-A-95/21256
- WO-A-98/23755
- FR-A- 2 737 500
- DATABASE EMBLHTG SEQ ID NO: HSAC64 Clone humain BAC RG083M05 de 7q21-7q22, 14 février 1997 PAULEY A: "The sequence of H. sapiens BAC clone RG083M05" XP002090413
- DATABASE EMBLHUM2 SEQ ID HSU85196 Homo sapiens BAC378, séquence complète, 27 mai 1997 BOYSEN C ET AL.: "Analysis of the 1.1-Mb human alpha/delta T-cell receptor locus with bacterial artificial chromosome clones" XP002090414
- DATABASE EMBLEMEST11 SEQ ID HSZZ32436 AC AA327384 EST30710 Colon I Homo sapiens cDNA 5', 18 avril 1997 ADAMS M D ET AL.: "Initial assessment of human gene diversity and expression patterns based upon 83 million nucleotides of cDNA sequence" XP002059257 & NATURE., vol. 377supp, 28 septembre 1995, pages 3-174, LONDON GB
- G. LA MANTIA ET AL.: "Identification and characterization of novel human endogenous retroviral sequences preferentially expressed in undifferentiated embryonal carcinoma cells" NUCLEIC ACIDS RESEARCH, vol. 19, no. 7, 1991, pages 1513-1520, XP002059255 OXFORD GB
- H. PERRON ET AL.: "Molecular identification of a novel retrovirus repeatedly isolated from patients with multiple sclerosis" PROCEEDINGS OF THE NATIONAL ACADEMY OF SCIENCES OF USA, vol. 94, no. 14, 8 juillet 1997, pages 7583-7588, XP002059256 WASHINGTON US

## Description

La Sclérose en Plaques (SEP) est une maladie démyélinisante du système nerveux central (SNC) dont la cause complète reste encore inconnue.

De nombreux travaux ont étayé l'hypothèse d'une étiologie virale de la maladie, mais aucun des virus connus testés ne s'est avéré être l'agent causal recherché: une revue des virus recherchés depuis des années dans la SEP a été faite par E. Norrby et R.T. Johnson.

Récemment, un rétrovirus, différent des rétrovirus humains connus a été isolé chez des patients atteints de SEP. Les auteurs ont aussi pu montrer que ce rétrovirus pouvait être transmis in vitro, que des patients atteints de SEP produisaient des anticorps susceptibles de reconnaitre des protéines associées à l'infection des cellules leptoméningées par ce rétrovirus, et que l'expression de ce dernier pouvait être fortement stimulée par les gènes immédiats-précoces de certains herpesvirus.

Tous ces résultats plaident en faveur du rôle dans la SEP d'au moins un rétrovirus inconnu ou d'un virus ayant une activité transcriptase inverse (RT) détectable selon la méthode publiée par H. Perron et qualifiée d'activité "RT de type LM7".

Les travaux de la Demanderesse ont permis d'obtenir deux lignées continues de cellules infectées par des isolats naturels provenant de deux patients différents atteints de SEP, par un procédé de culture tel que décrit dans le document WO-A-93 20188. Ces deux lignées dérivées de cellules de plexus-choroïdes humains, dénommées LM7PC et PLI-2 ont été déposées à l'E.C.A.C.C. respectivement le 22 juillet 1992 et le 8 janvier 1993, sous les numéros 92 072201 et 93 010817, conformément aux dispositions du Traité de Budapest. Par ailleurs, les isolats viraux possédant une activité RT de type LM7 ont également été déposés à l'E.C.A.C.C. sous la dénomination globale de "souches". La "souche" ou isolat hébergé par la lignée PLI-2, dénommée POL-2, a été déposée auprès de l'E.C.A.C.C. le 22 juillet 1992 sous le n° V92072202. La "souche" ou isolat hébergé par la lignée LM7PC, dénommée MS7PG, a été déposée auprès de l'E.C.A.C.C. le 8 janvier 1993 sous le n° V93010816.

A partir des cultures et des isolats précités, caractérisés par des critères biologiques et morphologiques, on s'est ensuite attaché à caractériser le matériel nucléique associé aux particules virales produites dans ces cultures.

Les portions de génome déjà caractérisées ont été utilisées pour mettre au point des tests de détection moléculaire du génome viral et des tests immunosérologiques, utilisant les séquences d'aminoacides codées par les séquences nucléotidiques du génome viral, pour détecter la réponse immunitaire dirigée contre des épitopes associés à l'infection et/ou l'expression virale.

Ces outils ont déjà permis de confirmer une association entre la SEP et l'expression des séquences identifiées dans les brevets cités plus loin. Cependant, le système viral découvert par la Demanderesse, s'apparente à un système rétroviral complexe. En effet, les séquences retrouvées encapsidées dans les particules virales extracellulaires produites par les différentes cultures de cellules de patients atteints de SEP, montrent clairement qu'il y a co-encapsidation de génomes rétroviraux apparentés, mais différents du génome rétroviral "sauvage" qui produit les particules virales infectantes. Ce phénomène a été observé entre des rétrovirus réplicatifs et des rétrovirus endogènes appartenant à la même famille, voire même hétérologues. La notion de rétrovirus endogène est très importante dans le contexte de notre découverte car, dans le cas de MSRV-1, on a observé que des séquences rétrovirales endogènes comprenant des séquences homologues au génome MSRV-1, existent dans l'ADN humain normal. L'existence d'éléments rétroviraux endogènes (ERV) apparentés à MSRV-1 par tout ou partie de leur génome, explique le fait que l'expression du rétrovirus MSRV-1 dans les cellules humaines puisse interagir avec des séquences endogènes proches. Ces interactions sont retrouvées dans le cas de rétrovirus endogènes pathogènes et/ou infectieux (par exemple certaines souches écotropes du Murine Leukaemia virus), dans le cas de rétrovirus exogènes dont la séquence nucléotidique peut être retrouvée partiellement ou en totalité, sous forme d'ERVs, dans le génome de l'animal hôte (ex. virus exogène de la tumeur mammaire de la souris transmis par le lait). Ces interactions consistent principalement en (i) une transactivation ou co-activation d'ERVs par le rétrovirus réplicatif, (ii) une encapsidation "illégitime" d'ARN apparentés d'ERVS, ou d'ERVs -voire d'ARN cellulaires- possédant simplement des séquences d'encapsidation compatibles, dans les particules rétrovirales produites par l'expression de la souche réplicative, parfois transmissibles et parfois avec une pathogénicité propre, et (iii) des recombinaisons plus ou moins importantes entre les génomes co-encapsidés, notamment dans les phases de transcription inverse, qui conduisent à la formation de génomes hybrides, parfois transmissibles et parfois avec une pathogénicité propre.

Ainsi, (i) différentes séquences apparentées à MSRV-1 ont été retrouvées dans les particules virales purifiées; (ii) l'analyse moléculaire des différentes régions du génome rétroviral MSRV-1 doit être faite en analysant systématiquement les séquences co-encapsidées, interférantes et/ou recombinées qui sont générées par l'infection et/ou l'expression de MSRV-1, de plus, certains clones peuvent avoir des parties de séquences défectives produites par la réplication rétrovirale et les erreurs de matrice et/ou de transcription de la transcriptase inverse; (iii) les familles de séquences apparentées à une même région génomique rétrovirale sont les supports d'une détection diagnostique globale qui peut être optimisée par l'identification de régions invariables parmi les clones exprimés et par l'identification de trames de lectures responsables de la production de polypeptides antigéniques et/ou pathogènes qui peuvent n'être produits que par une partie, voire un seul, des clones exprimés et dans ces conditions, l'analyse systématique des clones exprimés dans une région d'un gène donné permet d'évaluer la fréquence de variation et/ou de recombinaison du génome MSRV-1 dans cette région et de définir les séquences optimales pour les applications, notamment diagnostiques; (iv) la pathologie provoquée par un rétrovirus tel que MSRV-1 peut être un effet direct de son expression et des protéines ou peptides produits de ce fait, mais aussi un effet de l'activation, de l'encapsidation, de la recombinaison de génomes apparentés ou hétérologues et des protéines ou peptides produits de ces faits ; ainsi ces génomes associés à l'expression de et/ou l'infection par MSRV-1 sont-ils une partie intégrante de la pathogénicité potentielle de ce virus et donc, constituent des supports de détection diagnostique et des cibles thérapeutiques particulières. De même, tout agent associé à, ou, co-facteur de ces interactions responsables de la pathogénie en cause, tel que MSRV-2 ou le facteur gliotoxique décrit dans la demande de brevet publiée sous le N° FR-2 716 198, peut participer à l'élaboration d'une stratégie globale et très efficace de diagnostic, de pronostic, de suivi thérapeutique et/ou de thérapeutique intégrée de la SEP notamment, mais aussi de toute autre maladie associée aux mêmes agents.

Dans ce contexte, on a fait une découverte parallèle dans une autre maladie autoimmune, la polyarthrite rhumatoïde (PR), qui a été décrite dans la demande de brevet français déposée sous le N°95 02960. Cette découverte montre que, en appliquant des approches méthodologiques similaires à celles qui furent utilisées dans les travaux de la Demanderesse sur la SEP, on a pu identifier un rétrovirus exprimé dans la PR qui partage les séquences décrites pour MSRV-1 dans la SEP et aussi, la co-existence d'une séquence associée MSRV-2 également décrite dans la SEP. En ce qui concerne MSRV-1, les séquences détectées communément dans la SEP et la PR, concernent les gènes *pol* et *gag*. En l'état actuel des connaissances, on peut associer les séquences *gag* et *pol* décrites aux souches MSRV-1 exprimées dans ces deux maladies.

La présente demande de brevet a pour objet différents résultats, supplémentaires par rapport à ceux déjà protégés par les demandes de brevet français :
- N° 92 04322 du 03.04.1992, publiée sous le N° 2 689 519;
- N° 92 13447 du 03.11.1992, publiée sous le N° 2 689 521;
- N° 92 13443 du 03.11.1992, publiée sous le N° 2 689 520;
- N° 94 01529 du 04.02.1994, publiée sous le N° 2 715 936;
- N° 94 01531 du 04.02.1994, publiée sous le N° 2 715 939;
- N° 94 01530 du 04.02.1994, publiée sous le N° 2 715 936;
- N° 94 01532 du 04.02.1994, publiée sous le N° 2 715 937;
- N° 94 14322 du 24.11.1994, publiée sous le N° 2 727 428;
- N° 94 15810 du 23.12.1994, publiée sous le N° 2 728 585;
et
- la demande de brevet WO-97/06260.

La présente invention concerne tout d'abord un matériel nucléique, qui peut consister en un matériel rétroviral, à l'état isolé ou purifié, pouvant être appréhendé ou caractérisé de différentes manières :
- il comprend une séquence nucléotidique choisie dans le groupe qui consiste en (i) les séquences SEQ ID NO: 114, SEQ ID NO: 117, SEQ ID NO: 120 ; (ii) les séquences complémentaires aux séquences (i) ; et (iii) les séquences équivalentes aux séquences (i) ou (ii), en particulier les séquences présentant pour toute suite de 100 monomères contigus, au moins 50 %, et préférentiellement au moins 70 % d'homologie avec respectivement les séquences (i) ou (ii);
- il code pour un polypeptide présentant, pour toute suite contigue d'au moins 30 acides aminéés, au moins 50 %, et de préférence au moins 70 % d'homologie, avec une séquence peptidique choisie dans le groupe qui consiste en SEQ ID N°115, SEQ ID NO: 118, SEQ ID NO: 121;
- son gène env comprend une séquence nucléotidique identique ou équivalente à une séquence choisie dans le groupe qui consiste en SEQ ID NO: 117, et ses séquences complémentaires;
- son gène env comprend une séquence nucléotidique qui commence au nucléotide 1 de SEQ ID NO: 117 et finit au nucléotide au nucléotide 233 de SEQ ID NO: 114;
- son gène env code pour un polypeptide présentant, pour toute suite contigue d'au moins 30 acides aminéés, au moins 50 %, et de préférence au moins 70 % d'homologie, avec la séquence SEQ ID NO:°118;
- la région U3R de son LTR 3' comprend une séquence nucléotidique qui se termine au nucléotide 617 de SEQ ID NO: 114;
- la région RU5 de son LTR 5' comprend une séquence nucléotidique qui commence au nucléotide 755 de SEQ ID NO: 120
- un matériel nucléique rétroviral comprenant une séquence qui commence au nucléotide 755 de SEQ ID NO: 120 et qui se termine au nucléotide 617 de SEQ ID NO: 114;
- le matériel nucléique rétroviral tel que défini précédemment est en particulier associé à au moins une maladie auto-immune telle que la sclérose en plaques ou la polyarthrite rhumatoïde.

L'invention concerne aussi un fragment nucléotidique qui répond au moins à l'une des définitions suivantes :
- il comprend ou consiste en une séquence nucléotidique choisie dans le groupe qui consiste en (i) les séquences SEQ ID NO: 114, SEQ ID NO: 117, SEQ ID NO: 120; (ii) les séquences complémentaires des séquences (i) ; et (iii) les séquences équivalentes aux séquences (i) ou (ii), en particulier les séquences présentant pour toute suite de 100 monomères contigus, au moins 50 %, et préférentiellement au moins 70 % d'homologie avec respectivement les séquences (i) ou (ii);
- il comprend ou consiste en une séquence nucléotidique codant pour un polypeptide présentant, pour toute suite contigue d'au moins 30 acides aminéés, au moins 50 %, et de préférence au moins 70 % d'homologie, avec une séquence peptidique choisie dans le groupe qui consiste en SEQ ID N°115, SEQ ID NO: 118, SEQ ID NO: 121.

D'autres objets de la présente invention sont les suivants :
- une sonde nucléique pour la détection d'un rétrovirus associé à la sclérose en plaques et/ou la polyarthrite rhumatoïde, susceptible de s'hybrider spécifiquement sur tout fragment précédemment défini et appartenant au génome dudit rétrovirus; elle possède avantageusement de 10 à 100 nucléotides, de préférence de 10 à 30 nucléotides;
- une amorce pour l'amplification par polymérisation d'un ARN ou d'un ADN d'un rétrovirus associé à la sclérose en plaques et/ou la polyarthrite rhumatoïde, qui comprend une séquence nucléotidique identique ou équivalente à au moins une partie de la séquence nucléotidique d'un fragment défini précédemment, notamment une séquence nucléotidique présentant pour toute suite de 10 monomères contigus, au moins 50 %, de préférence au moins 70 % d'homologie avec au moins ladite partie dudit fragment ; de préférence la séquence nucléotidique d'une amorce de l'invention est choisie parmi SEQ ID NO: 116 et SEQ ID NO: 119;
- un ARN ou ADN, et notamment vecteur de réplication et/ou d'expression, comprenant un fragment génomique du matériel nucléique ou un fragment défini précédemment;
- un peptide codé par tout cadre de lecture ouvert appartenant à un fragment nucléotidique défini précédemment, notamment un polypeptide, par exemple oligopeptide formant ou comprenant un déterminant antigénique reconnu par des sera de patients infectés par le virus MSRV-1, et/ou chez lesquels le virus MSRV-1 a été réactivé; un peptide préférentiel comprend une séquence identique, partiellement ou totalement, ou équivalente à une séquence choisie parmi SEQ ID N°115, SEQ ID NO: 118, SEQ ID NO: 121;
- une composition diagnostique, prophylactique, ou thérapeutique, notamment pour inhiber l'expression d'au moins un rétrovirus associé à la sclérose en plaques et/ou à la polyarthrite rhumatoïde, comprenant un fragment nucléotidique défini précédemment;
- un procédé pour détecter un rétrovirus associé à la sclérose en plaques et/ou à la polyarthrite rhumatoïde, dans un échantillon biologique, comprenant les étapes consistant à mettre en contact un ARN et/ou un ADN présumé appartenir ou provenant dudit rétrovirus, ou leur ARN et/ou ADN complémentaire, avec une composition comprenant un fragment nucléotidique défini ci-dessus.

Avant de détailler l'invention, différents termes utilisés dans la description et les revendications sont à présent définis :
- par souche ou isolat, on entend toute fraction biologique infectante et/ou pathogène, contenant par exemple des virus et/ou des bactéries et/ou des parasites, générant un pouvoir pathogène et/ou antigénique, hébergée par une culture ou un hôte vivant ; à titre d'exemple, une souche virale selon la définition précédente peut contenir un agent co-infectant, par exemple un protiste pathogène,
- le terme "MSRV" utilisé dans la présente description désigne tout agent pathogène et/ou infectant, associé à la SEP, notamment une espèce virale, les souches atténuées de ladite espèce virale, ou les particules défectives interférentes ou contenant des génomes co-encapsidés ou encore des génomes recombinés avec une partie du génome MSRV-1, dérivées de cette espèce. Il est connu que les virus et particulièrement les virus contenant de l'ARN ont une variabilité, consécutive notamment à des taux relativement élevés de mutation spontanée, dont il sera tenu compte ci-après pour définir la notion d'équivalence,
- par virus humain, on entend un virus susceptible d'infecter ou d'être hébergé par l'être humain,
- compte tenu de toutes les variations et/ou recombinaison naturelles ou induites, pouvant être rencontrées dans la pratique de la présente invention, les objets de cette dernière, définis ci-dessus et dans les revendications, ont été exprimés en comprenant les équivalents ou dérivés des différents matériels biologiques définis ci-après, notamment des séquences homologues nucléotidiques ou peptidiques,
- le variant d'un virus ou d'un agent pathogène et/ou infectant selon l'invention, comprend au moins un antigène reconnu par au moins un anticorps dirigé contre au moins un antigène correspondant dudit virus et/ou dudit agent pathogène et/ou infectant, et/ou un génome dont toute partie est détectée par au moins une sonde d'hybridation, et/ou au moins une amorce d'amplification nucléotidique spécifique dudit virus et/ou agent pathogène et/ou infectant, dans des conditions d'hybridation déterminées bien connues de l'homme de l'art,
- selon l'invention, un fragment nucléotidique ou un oligonucléotide ou un polynucléotide est un enchaînement de monomères, ou un biopolymère, caractérisé par la séquence informationnelle des acides nucléiques naturels, susceptible de s'hybrider à tout autre fragment nucléotidique dans des conditions prédéterminées, l'enchaînement pouvant contenir des monomères de structures chimiques différentes et être obtenu à partir d'une molécule d'acide nucléique naturelle et/ou par recombinaison génétique et/ou par synthèse chimique ; un fragment nucléotidique peut être identique à un fragment génomique du virus MSRV-1 considéré par la présente invention, notamment un gène de ce dernier, par exemple pol ou env dans le cas dudit virus ;
- ainsi un monomère peut être un nucléotide naturel d'acide nucléique, dont les éléments constitutifs sont un sucre, un groupement phosphate et une base azotée; dans l'ARN le sucre est le ribose, dans l'ADN le sucre est le désoxy-2-ribose; selon qu'il s'agit de l'ADN ou l'ARN, la base azotée est choisie parmi l'adénine, la guanine, l'uracile, la cytosine, la thymine; ou le nucléotide peut être modifié dans l'un au moins des trois éléments constitutifs ; à titre d'exemple, la modification peut intervenir au niveau des bases, générant des bases modifiées telles que l'inosine, la méthyl-5-désoxycytidine, la désoxyuridine, la diméthylaminéo-5-désoxyuridine, la diaminéo-2,6-purine, la bromo-5-désoxyuridine et toute autre base modifiée favorisant l'hybridation; au niveau du sucre, la modification peut consister dans le remplacement d'au moins un désoxyribose par un polyamide, et au niveau du groupement phosphate, la modification peut consister dans son remplacement par des esters, notamment choisis parmi les esters de diphosphate, d'alkyl et arylphosphonate et de phosphorothioate,
- par "séquence informationnelle", on entend toute suite ordonnée de monomères, dont la nature chimique et l'ordre dans un sens de référence, constituent ou non une information fonctionnelle de même qualité que celle des acides nucléiques naturels,
- par hybridation, on entend le processus au cours duquel, dans des conditions opératoires appropriées, deux fragments nucléotidiques, ayant des séquences suffisamment complémentaires, s'apparient pour former une structure complexe, notamment double ou triple, de préférence sous forme d'hélice,
- une sonde comprend un fragment nucléotidique synthétisé par voie chimique ou obtenu par digestion ou coupure enzymatique d'un fragment nucléotidique plus long, comprenant au moins six monomères, avantageusement de 10 à 100 monomères, de préférence 10 à 30 monomères, et possédant une spécificité d'hybridation dans des conditions déterminées ; de préférence, une sonde possédant moins de 10 monomères n'est pas utilisée seule, mais l'est en présence d'autres sondes de taille aussi courte ou non ; dans certaines conditions particulières, il peut être utile d'utiliser des sondes de taille supérieure à 100 monomères ; une sonde peut notamment être utilisée à des fins de diagnostic et il s'agira par exemple de sondes de capture et/ou de détection,
- la sonde de capture peut être immobilisée sur un support solide par tout moyen approprié, c'est-à-dire directement ou indirectement, par exemple par covalence ou adsorption passive,
- la sonde de détection peut être marquée au moyen d'un marqueur choisi notamment parmi les isotopes radioactifs, des enzymes notamment choisis parmi la peroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine,
- les sondes utilisées à des fins de diagnostic de l'invention peuvent être mises en oeuvre dans toutes les techniques d'hybridation connues, et notamment les techniques dites "DOT-BLOT", "SOUTHERN BLOT", "NORTHERN BLOT" qui est une technique identique à la technique "SOUTHERN BLOT" mais qui utilise de l'ARN comme cible, la technique SANDWICH ; avantageusement, on utilise la technique SANDWICH dans la présente invention, comprenant une sonde de capture spécifique et/ou une sonde de détection spécifique, étant entendu que la sonde de capture et la sonde de détection doivent présenter une séquence nucléotidique au moins partiellement différente,
- toute sonde selon la présente invention peut s'hybrider in vivo ou in vitro sur l'ARN et/ou sur l'ADN, pour bloquer les phénomènes de réplication, notamment traduction et/ou transcription, et/ou pour dégrader ledit ADN et/ou ARN,
- une amorce est une sonde comprenant au moins six monomères, et avantageusement de 10 à 30 monomères, possédant une spécificité d'hybridation dans des conditions déterminées, pour l'initiation d'une polymérisation enzymatique, par exemple dans une technique d'amplification telle que la PCR (Polymerase Chain Reaction), dans un procédé d'élongation, tel que le séquençage, dans une méthode de transcription inverse ou analogue,
- deux séquences nucléotidiques ou peptidiques sont dites équivalentes ou dérivées l'une par rapport à l'autre, ou par rapport à une séquence de référence, si fonctionnellement les biopolymères correspondants peuvent jouer sensiblement le même rôle, sans être identiques, vis-à-vis de l'application ou utilisation considérée, ou dans la technique dans laquelle elles interviennent ; sont notamment équivalentes deux séquences obtenues du fait de la variabilité naturelle, notamment mutation spontanée de l'espèce à partir de laquelle elles ont été identifiées, ou induite, ainsi que deux séquences homologues, l'homologie étant définie ci-après,
- par "variabilité", on entend toute modification, spontanée ou induite d'une séquence, notamment par substitution, et/ou insertion, et/ou délétion de nucléotides et/ou de fragments nucléotidiques, et/ou extension et/ou racourcissement de la séquence à l'une au moins des extrémités; une variabilité non naturelle peut résulter des techniques de génie génétique utilisées, par exemple du choix des amorces de synthèse dégénérées ou non, retenues pour amplifier un acide nucléique; cette variabilité peut se traduire par des modifications de toute séquence de départ, considérée comme référence, et pouvant être exprimées par un degré d'homologie par rapport à ladite séquence de référence,
- l'homologie caractérise le degré d'identité de deux fragments nucléotidiques ou peptidiques comparés ; elle se mesure par le pourcentage d'identité qui est notamment déterminé par comparaison directe de séquences nucléoditiques ou peptidiques, par rapport à des séquences nucléotidiques ou peptidiques de référence,
- tout fragment nucléotidique est dit équivalent ou dérivé d'un fragment de référence, s'il présente une séquence nucléotidique équivalente à la séquence du fragment de référence ; selon la définition précédente, sont notamment équivalents à un fragment nucléotidique de référence :
   (a) tout fragment susceptible de s'hybrider au moins partiellement avec le complément du fragment de référence,
   (b) tout fragment dont l'alignement avec le fragment de référence conduit à mettre en évidence des bases contigues identiques, en nombre plus important qu'avec tout autre fragment provenant d'un autre groupe taxonomique,
   (c) tout fragment résultant ou pouvant résulter de la variabilité naturelle de l'espèce, à partir de laquelle il est obtenu,
   (d) tout fragment pouvant résulter des techniques de génie génétique appliquées au fragment de référence,
   (e) tout fragment, comportant au moins huit nucléotides contigus, codant pour un peptide homologue ou identique au peptide codé par le fragment de référence,
   (f) tout fragment différent du fragment de référence, par insertion, délétion, substitution d'au moins un monomère, extension, ou raccourcissement à l'une au moins de ses extrémités ; par exemple, tout fragment correspondant au fragment de référence, flanqué à l'une au moins de ses extrémités par une séquence nucléotidique ne codant pas pour un polypeptide,
- par polypeptide, on entend notamment tout peptide d'au moins deux acides aminéés, notamment oligopeptide, protéine, extrait, séparé, ou substantiellement isolé ou synthétisé, par l'intervention de la main de l'homme, notamment ceux obtenus par synthèse chimique, ou par expression dans un organisme recombinant,
- par polypeptide codé de manière partielle par un fragment nucléotidique, on entend un polypeptide présentant au moins trois acides aminéés codés par au moins neuf monomères contigus compris dans ledit fragment nucléotidique,
- un acide aminéé est dit analogue à un autre acide aminée, lorsque leur caractéristiques physico-chimiques respectives, telles que polarité, hydrophobicité, et/ou basicité, et/ou acidité, et/ou neutralité, sont sensiblement les mêmes ; ainsi, une leucine est analogue à une isoleucine.
- tout polypeptide est dit équivalent ou dérivé d'un polypeptide de référence, si les polypeptides comparés ont sensiblement les mêmes propriétés, et notamment les mêmes propriétés antigéniques, immunologiques, enzymologiques et/ou de reconnaissance moléculaire ; est notamment équivalent à un polypeptide de référence :
   (a) tout polypeptide possédant une séquence dont au moins un acide aminéé a été substitué par un acide aminéé analogue,
   (b) tout polypeptide ayant une séquence peptidique équivalente, obtenue par variation naturelle ou induite dudit polypeptide de référence, et/ou du fragment nucléotidique codant pour ledit polypeptide,
   (c) un mimotope dudit polypeptide de référence,
   (d) tout polypeptide dans la séquence duquel un ou plusieurs acides aminéés de la série L sont remplacés par un acide aminéé de la série D, et vice versa,
   (e) tout polypeptide dans la séquence duquel on a introduit une modification des chaînes latérales des acides aminéés, telle que par exemple une acétylation des fonctions aminées, une carboxylation des fonctions thiol, une estérification des fonctions carboxyliques,
   (f) tout polypeptide dans la séquence duquel une ou des liaisons peptidiques ont été modifiées, comme par exemple les liaisons carba, rétro, inverso, rétro-inverso, réduites, et méthylène-oxy,
   (g) tout polypeptide dont au moins un antigène est reconnu par anticorps dirigé contre un polypeptide de référence,
- le pourcentage d'identité caractérisant l'homologie de deux fragments peptidiques comparés est selon la présente invention d'au moins 50% et de préférence au moins 70 %.

Etant donné qu'un virus possédant une activité enzymatique transcriptase inverse peut être génétiquement caractérisé aussi bien sous forme d'ARN que d'ADN, il sera fait mention aussi bien de l'ADN que de l'ARN viral pour caractériser les séquences relatives à un virus possédant une telle activité transcriptase inverse, dit MSRV-1 selon la présente description.

Les expressions d'ordre utilisées dans la présente description et les revendications, telles que "première séquence nucléotidique" ne sont pas retenues pour exprimer un ordre particulier, mais pour définir plus clairement l'invention.

Par détection d'une substance ou agent, on entend ci-après aussi bien une identification, qu'une quantification, ou une séparation ou isolement de ladite substance ou dudit agent.

L'invention sera mieux comprise à la lecture de la description détaillée qui va suivre faite en référence aux figures annexées dans lesquelles :

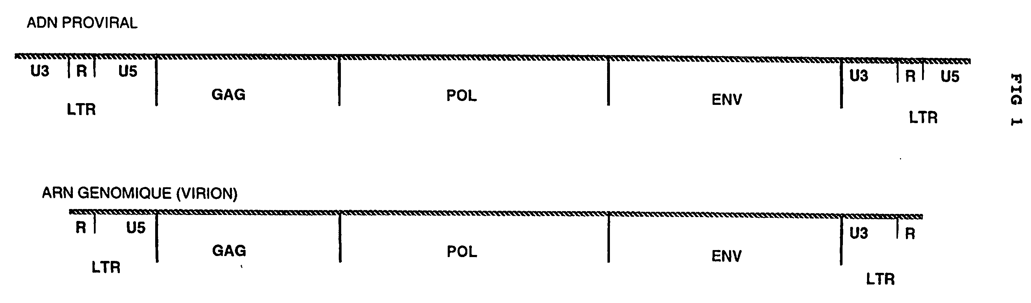

La Figure 1 représente la structure générale de l'ADN proviral et l'ARN génomique de MSRV-1.
La Figure 2 représente la séquence nucléotidique du clone dénommé CL6-5' (SEQ ID NO: 112) et trois trames de lecture potentielles en acides aminéés figurant sous la séquence nucléotidique.
La Figure 3 représente la séquence nucléotidique du clone dénommé CL6-3' (SEQ ID NO: 114) et trois trames de lecture potentielles en acides aminéés figurant sous la séquence nucléotidique.

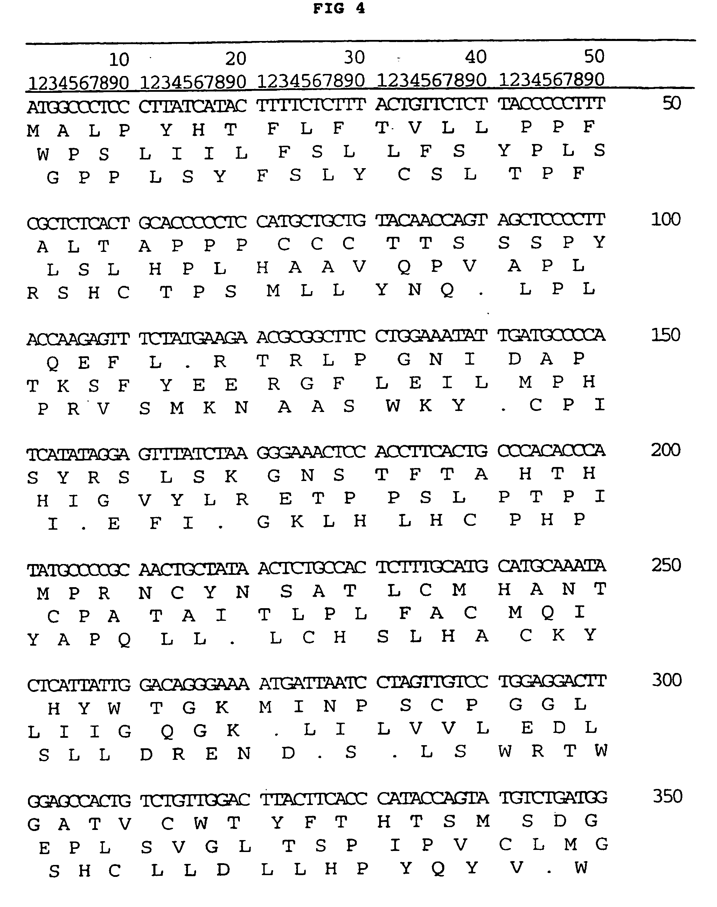

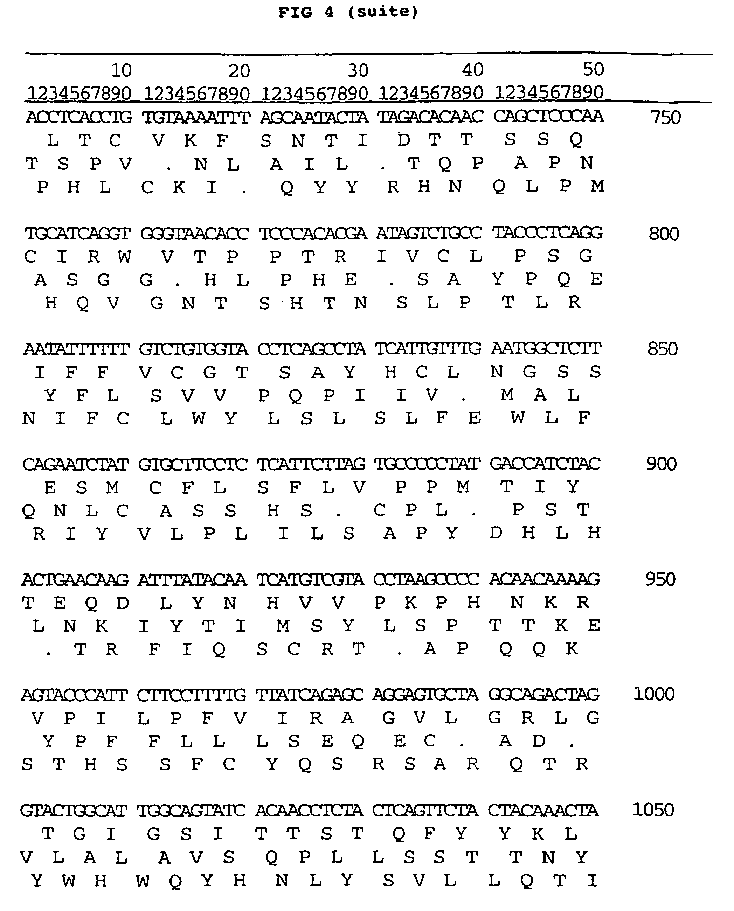

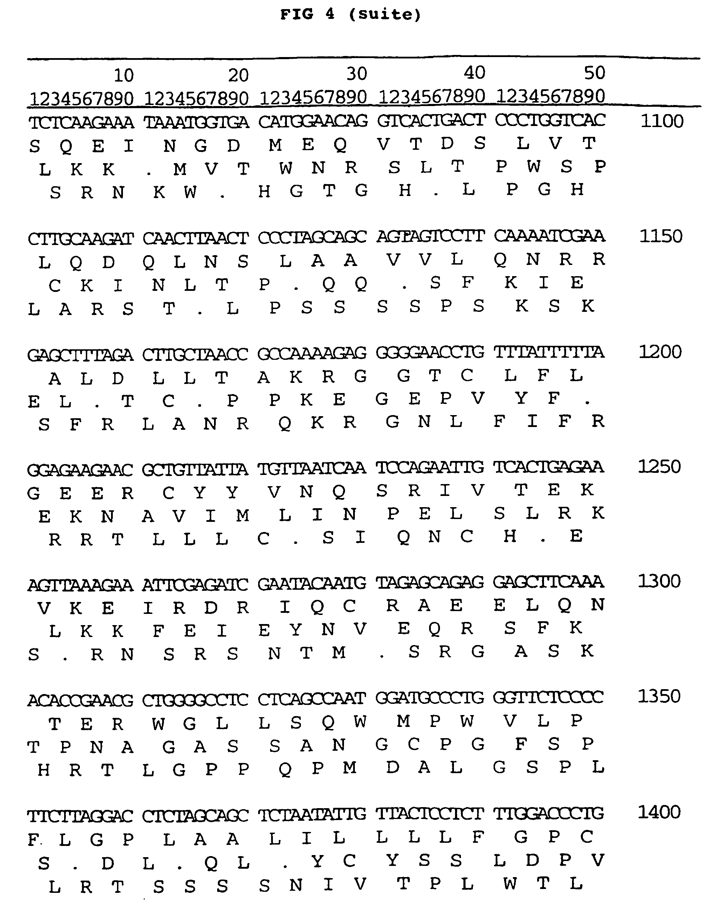

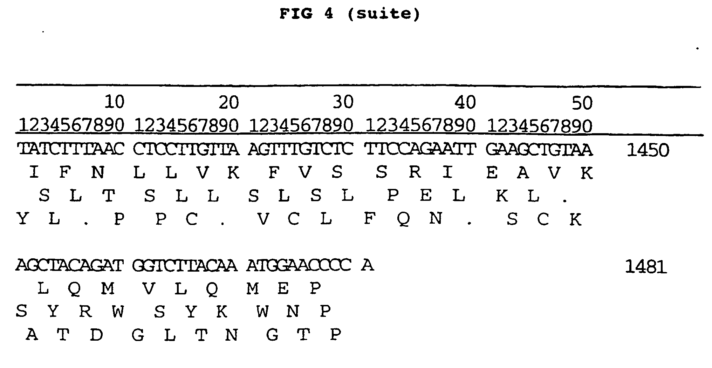

La Figure 4 représente la séquence nucléotidique du clone dénommé C15 (SEQ ID NO: 117) et trois trames de lecture potentielles en acides aminéés figurant sous la séquence nucléotidique.
La Figure 5 représente la séquence nucléotidique du clone dénommé 5M6 (SEQ ID NO: 120) et trois trames de lecture potentielles en acides aminéés figurant sous la séquence nucléotidique.
La Figure 6 représente la séquence nucléotidique du clone dénommé CL2 (SEQ ID NO: 130) et trois trames de lecture potentielles en acides aminéés figurant sous la séquence nucléotidique.

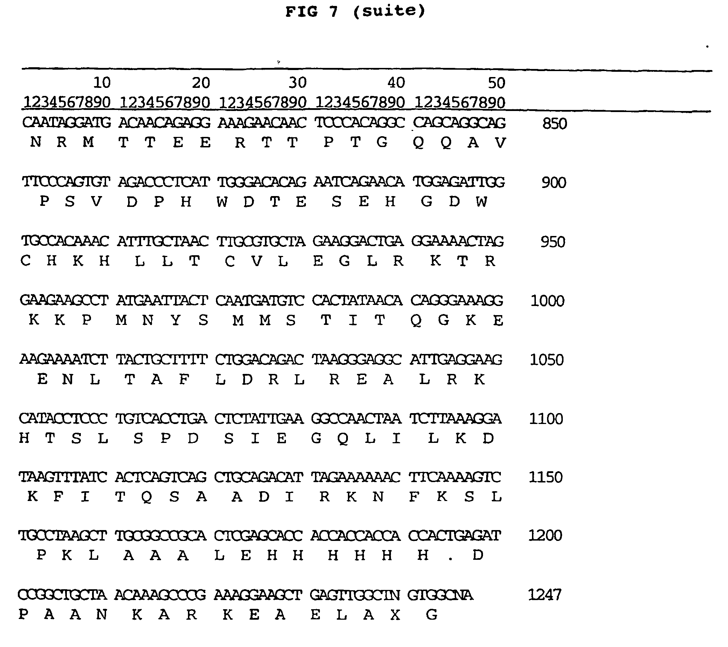

La Figure 7 représente trois trames de lecture potentielles en acides aminéés exprimées par pET28C-clone 2 et figurant sous la séquence nucléotidique.

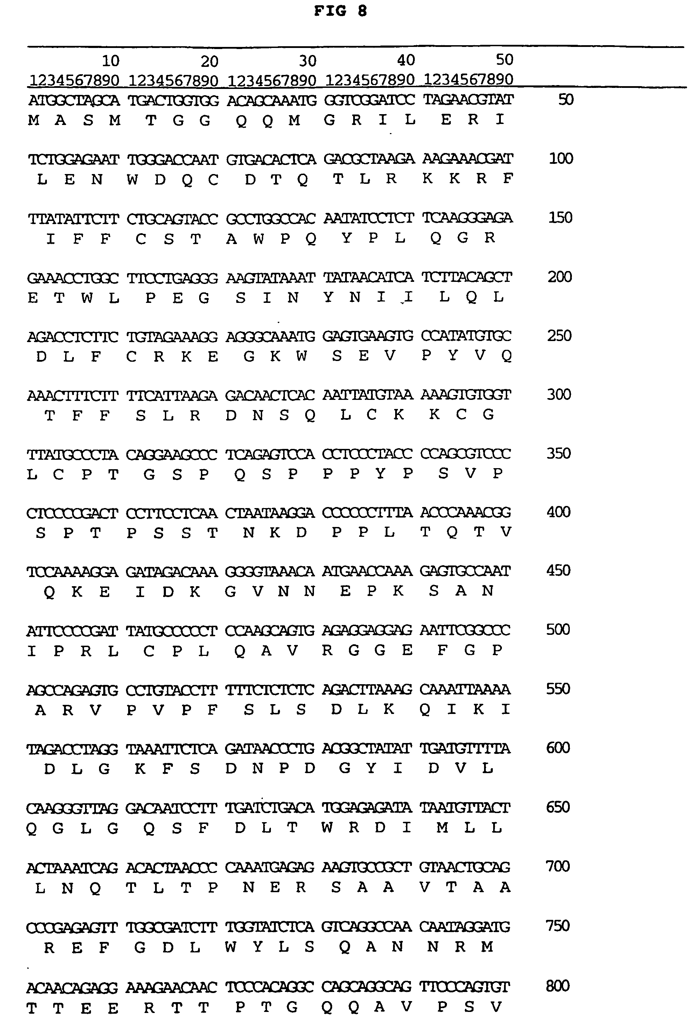

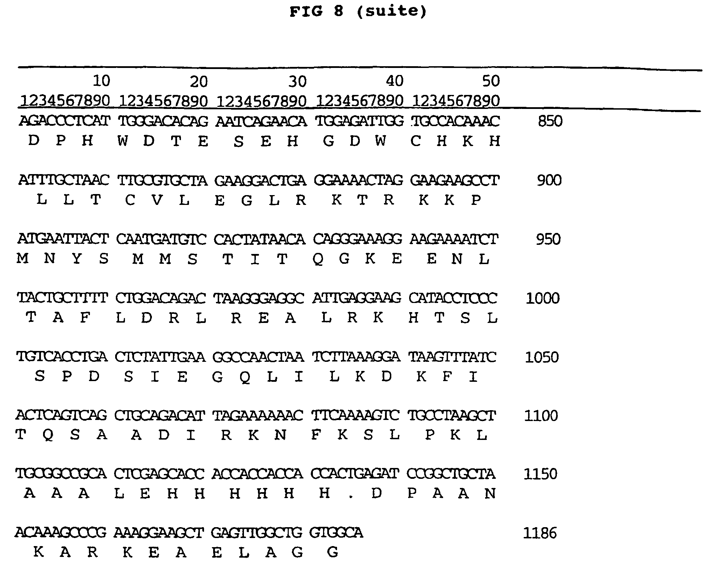

La Figure 8 représente trois trames de lecture potentielles en acides aminéés exprimées par pET21C-clone 2 et figurant sous la séquence nucléotidique.

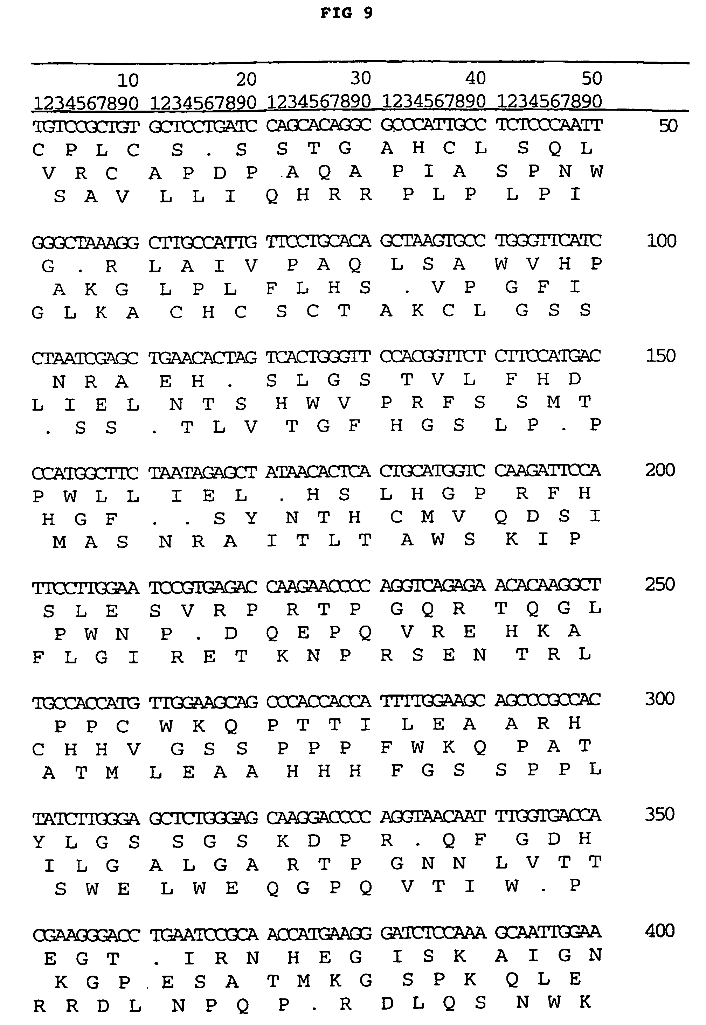

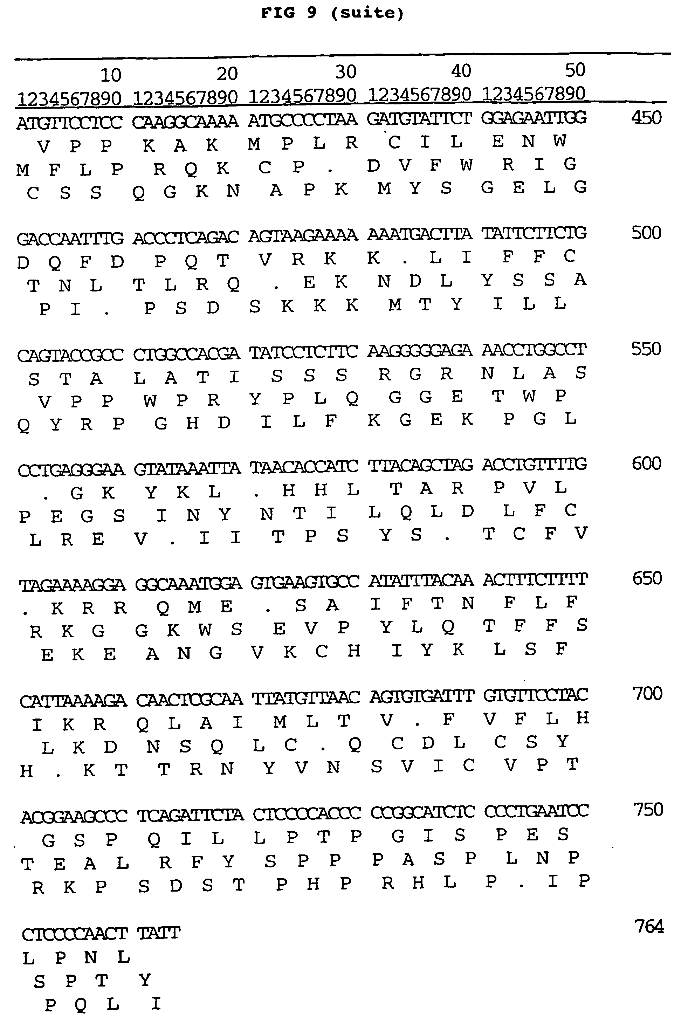

La Figure 9 représente la séquence nucléotidique du clone dénommé LB13 (SEQ ID NO: 141) et trois trames de lecture potentielles en acides aminéés figurant sous la séquence nucléotidique.

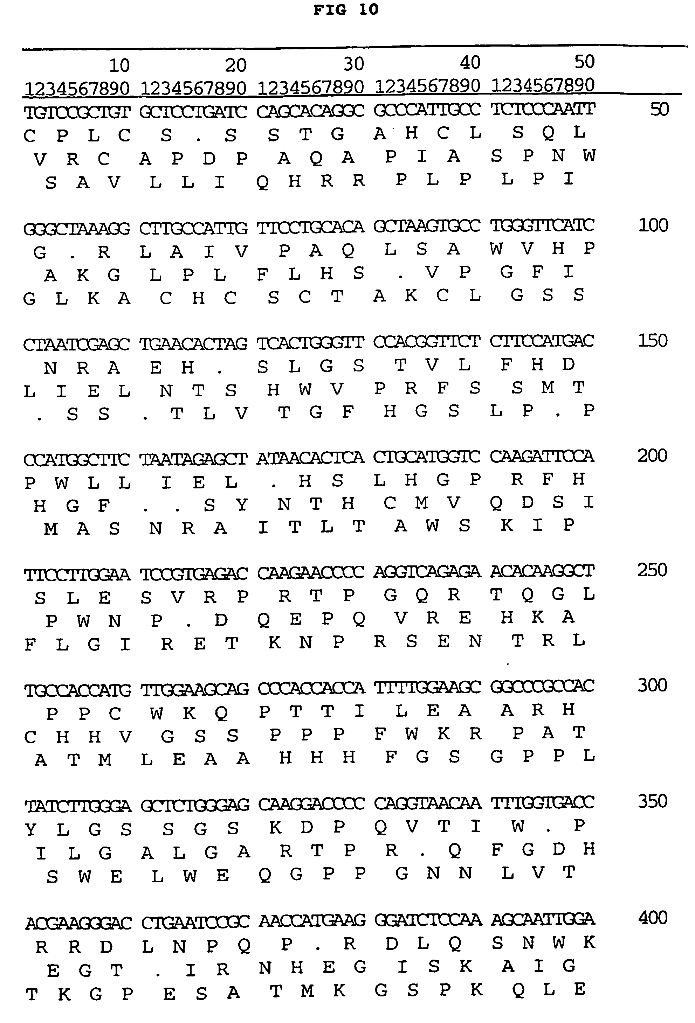

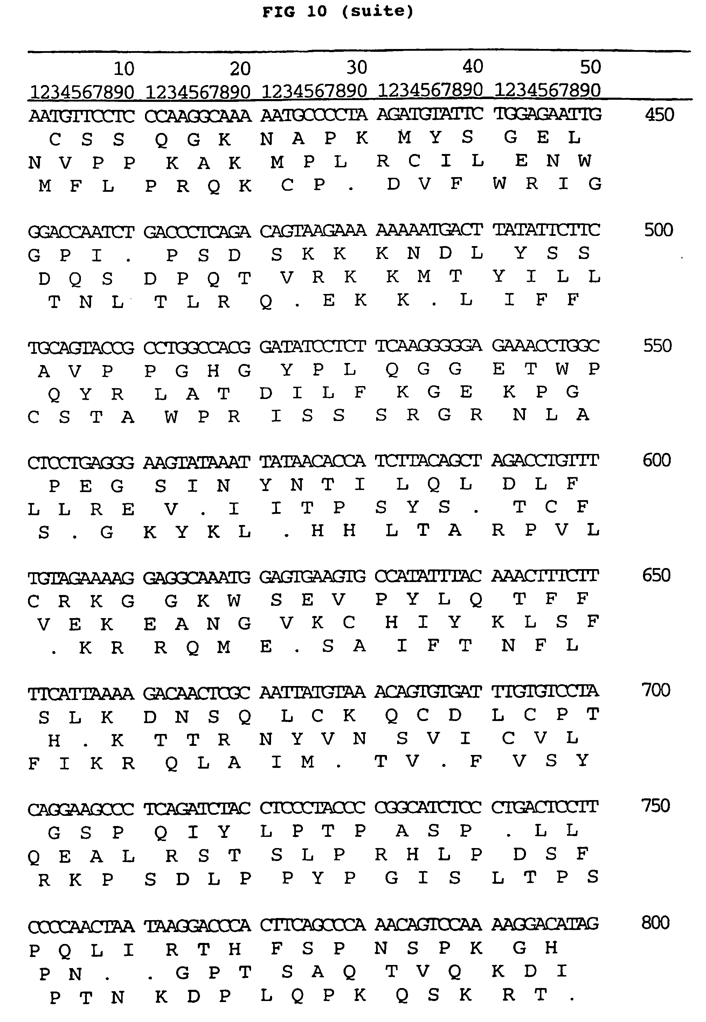

La Figure 10 représente la séquence nucléotidique du clone dénommé LA15 (SEQ ID NO: 142) et trois trames de lecture potentielles en acides aminéés figurant sous la séquence nucléotidique.
La Figure 11 représente la séquence nucléotidique du clone dénommé LB16 (SEQ ID NO: 124) et trois trames de lecture potentielles en acides aminéés figurant sous la séquence nucléotidique.
La Figure 12 représente l'activité promotrice exprimée en cpm/4min des séquences U3R sous clonées à partir de LTRs d'origines différentes dans le plasmide PCAT3. PCAT3 signifie plasmide seul, PCAT-PH74 signifie plasmide plus clone U3Rendogène exprimé dans le placenta, PCAT-c16 signifie plasmide plus clone U3R amplifié dans l'ARN d'un plasma SEP, PCAT-5M6 signifie plasmide plus région U3R amplifiée dans l'ADN cellulaire, "no plasmid" signifie absence de plasmide dans le test.
La Figure 13 représente les séquences MSRV1 env et LTR3'. Les flèches horizontales indiquent le début des régions env, U3 et R. Dans la région env : le peptide signal et la région immunosuppressive potentielle sont soulignés, les sites de glycosilation potentiels sont encadrés et les sites de clivage potentiels sont indiqués par des flèches verticales. Dans la région U3R : l'élément de régulation CAAT et la TATA Box sont soulignés, le site "cap" et le signal de polyadénylation sont aussi indiqués.

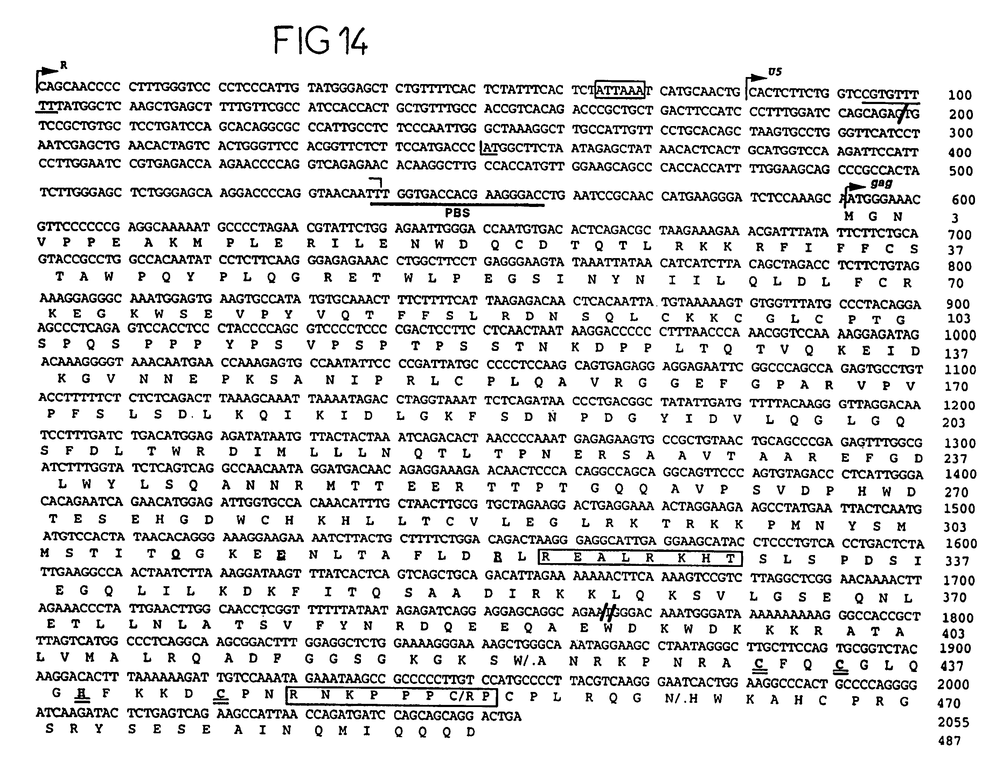

La Figure 14 représente une région LTR5' (RU5) suivie d'un site PBS (primer binding site) complémentaire du tARN Trp et d'un gène gag codant pour une protéine d'environ 487 acides aminés. Les acides aminés conservés dans la nucléocapside sont soulignés deux fois. Les acides aminés définissant la région de plus forte homologie dans la capside sont en gras et soulignés une fois. Les / dans la séquence en acides aminés indiquent des variations observées selon les clones et dans la séquence nucléotidique ils indiquent des sauts de trame dans certains clones. Les régions encadrées correspondent à des épitopes identifiés par analyse peptidique de la région C-terminale.
La Figure 15 représente la région intégrase de MSRV1, la séquence nucléotidique et la séquence en acides aminés déduite de la région intégrase correspondant au clone 87-23. Dans la Figure 15 // signifie un saut de trame qui a été supprimé pour restituer l'ORF potentiel. Les lettres en caractères gras soulignées représentent les acides aminés conservés des intégrases rétrovirales.
La Figure 16 décrit les séquences nucléotidique et peptidique du clone B13 (identique au clone FBd13 décrit dans des demandes antérieures) avec indication des ORFs et des codons stop représentés par un point. La région soulignée en gras représente le domaine immunosuppresseur potentiel. Le domaine souligné en simple représente le début du LTR3'.

### EXEMPLE 1: OBTENTION D'UNE REGION CL6-5' CODANT POUR L'EXTREMITE N-TERMINALE DE L'INTEGRASE ET D'UNE REGION CL6-3' CONTENANT LA SEQUENCE 3' TERMINALE DU GENOME MSRV-1

Une 3'RACE a été effectuée sur de l'ARN total extrait de plasma d'un patient atteint de SEP. Un plasma témoin sain, traité sous les mêmes conditions, a été utilisé comme contrôle négatif. La synthèse de cDNA a été réalisée avec une amorce oligo dT identifiée par SEQ ID NO: 68 (5' GAC TCG CTG CAG ATC GAT TTT TTT TTT TTT TTT T 3') et la transcriptase inverse "Expand™ RT" de Boehringer selon les conditions préconisées par la société. Une PCR a été effectuée avec l'enzyme Klentaq (Clontech) sous les conditions suivantes : 94°C 5 min puis 93°C 1 min, 58°C 1 min, 68°C 3 min pendant 40 cycles et 68°C pendant 8 min, avec un volume réactionnel final de 50 µl.
Amorces utilisées pour la PCR:
- amorce 5', identifiée par SEQ ID NO: 69
- amorce 3', identifiée par SEQ ID NO: 68

Une deuxième PCR dite "semi-nichée" a été réalisée avec une amorce 5' située à l'intérieur de la région déjà amplifiée. Cette deuxième PCR a été effectuée sous les mêmes conditions expérimentales que celles utilisées lors de la première PCR, en utilisant 10 µl du produit d'amplification issu de la première PCR.
Amorces utilisées pour la PCR semi-nichée:
- amorce 5', identifiée par SEQ ID NO: 70
- amorce 3', identifiée par SEQ ID NO: 68
Les amorces SEQ ID NO: 69 et SEQ ID NO: 70 sont spécifiques de la région pol de MSRV-1.

Un produit d'amplification de 1,9Kb a été obtenu pour le plasma de patient SEP. Le fragment correspondant n'a pas été observé pour le plasma témoin sain. Ce produit d'amplification a été cloné de la façon suivante :
l'ADN amplifié a été inséré dans un plasmide à l'aide du kit TA Cloning®. Les 2 µl de solution d'ADN ont été mélangés avec 5 µl d'eau distillée stérile, 1µl d'un tampon de ligation 10 fois concentré "10X LIGATION BUFFER", 2 µl de "pCR™ VECTOR" (25 ng/ml) et 1 µl de "T4 DNA LIGASE". Ce mélange a été incubé une nuit à 12°C. Les étapes suivantes ont été réalisées conformément aux instructions du kit TA Cloning® (Invitrogen). A la fin de la procédure, les colonies blanches de bactéries recombinantes (white) ont été repiquées pour être cultivées et permettre l'extraction des plasmides incorporés selon la procédure dite de "miniprep". La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA cloning kit®. La réaction préalable au séquençage a ensuite été effectuée selon la méthode préconisée pour l'utilisation du kit de séquençage "PRISM™ Ready Reaction AmpliTaq® FS, DyeDeoxy™ Terminator" (Applied Biosystems, ref. 402119) et le séquençage automatique a été réalisé sur les appareils 373 A et 377 Applied Biosystems, selon les instructions du fabricant.

Le clone obtenu, contient une région CL6-5'codant pour l'extrémité N terminale de l'intégrase et une région CL6-3', correspondant à la région 3' terminale de MSRV-1 et permettant de définir la fin de l'enveloppe (234 pb) et les régions U3, R (401 pb) du rétrovirus MSRV1.

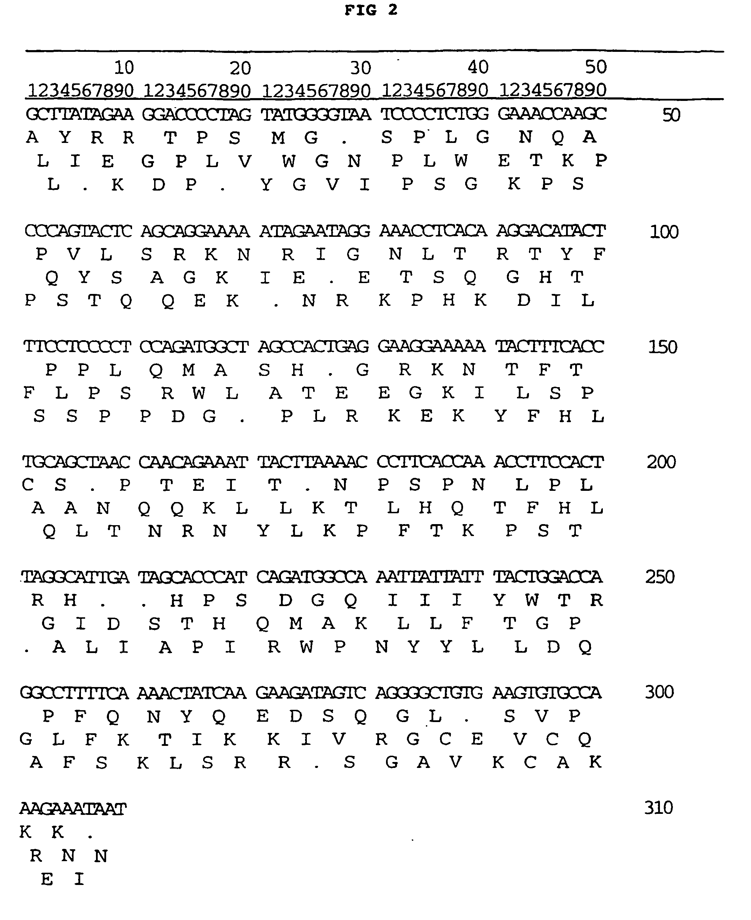

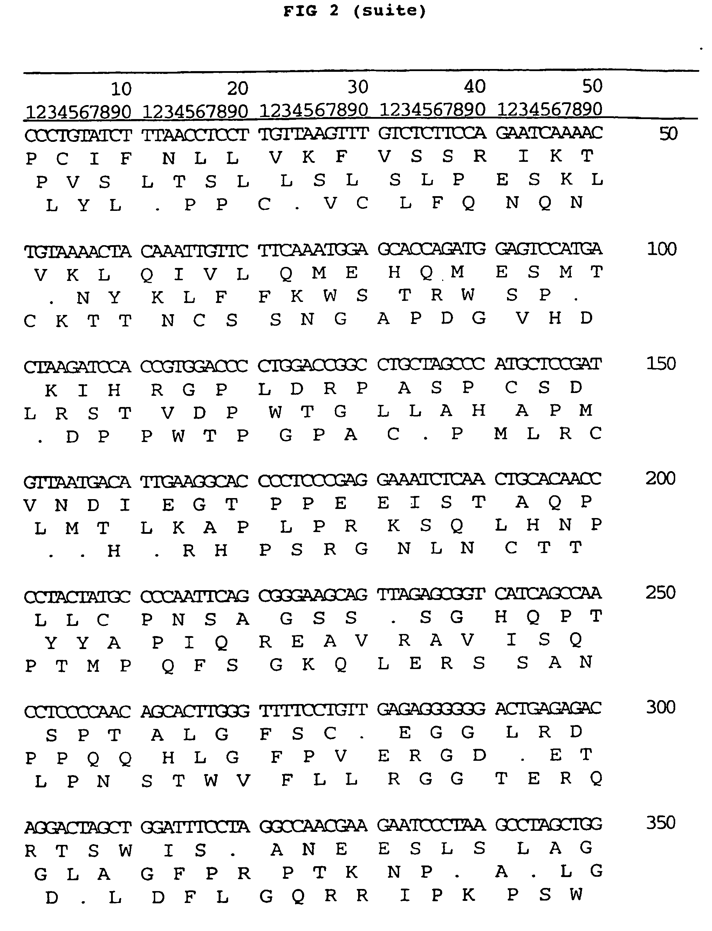

La région correspondant à l'extrémité N terminale de l'intégrase est représentée par sa séquence nucléotidique (SEQ ID NO: 112) dans la figure 2. Les trois trames de lecture potentielles sont présentées par leur séquence aminéoacide sous la séquence nucléotidique, et la séquence aminéoacide de l'extrémité N-terminale de l'intégrase est identifiée par SEQID NO: 113.

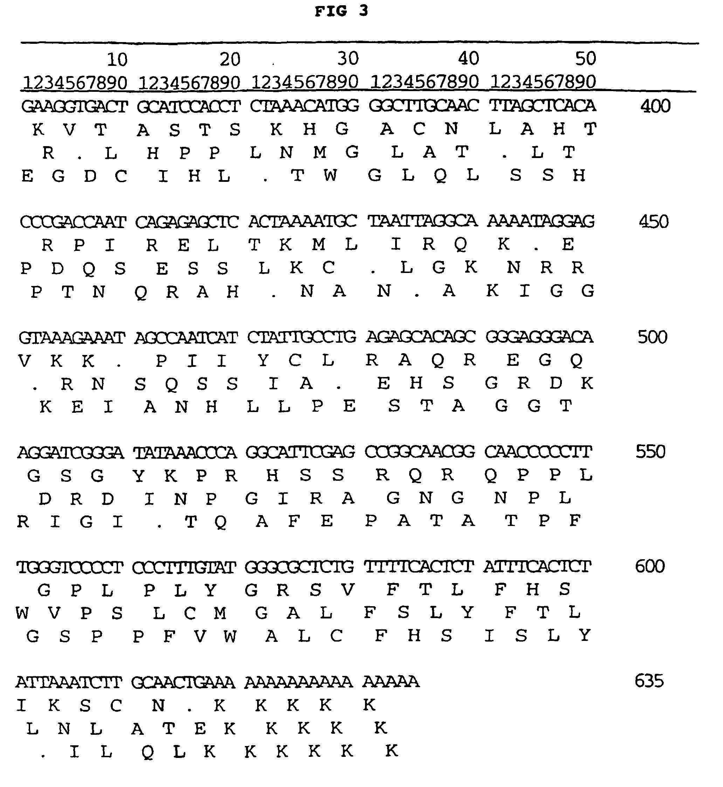

La région C16-3' est représentée par sa séquence nucléotidique (SEQ ID NO: 114) dans la figure 3. Les trois trames de lecture potentielles sont présentées par leur séquence aminéoacide sous la séquence nucléotidique. Une séquence aminéoacide correspondant à l'extrémité C-terminale de la protéine env de MSRV-1 est identifiée par SEQ ID NO: 115.

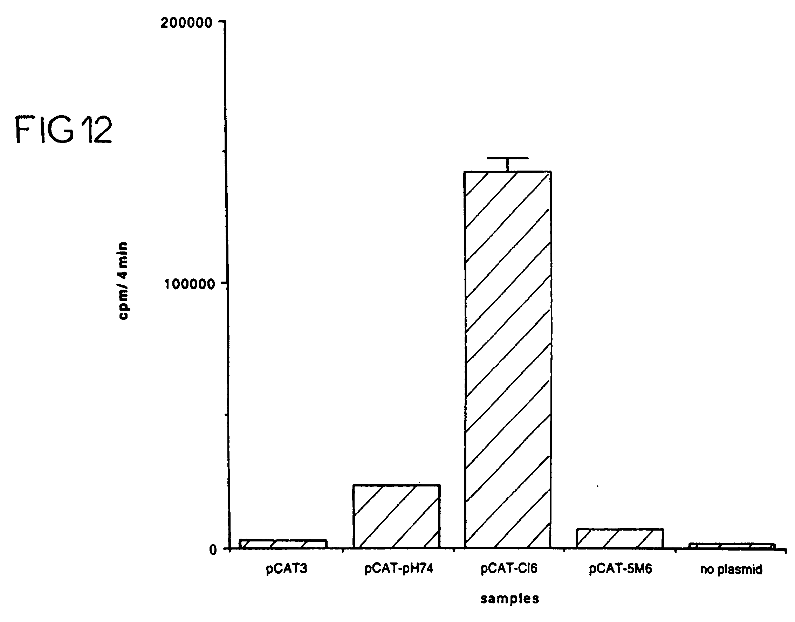

Afin d'évaluer l'activité promotrice du LTR obtenu à partir de clone 6 (c16) un test d'activité promotrice utilisant l'enzyme CAT (chloramphénicol acetyl transferase) a été effectué avec la région U3R correspondante. En parallèle un clone contenant la même région U3R d'ARN rétroviral endogène exprimé dans le placenta normal (PH74) et un clone (5M6) provenant d'ADN ont été testés. Le résultat présenté dans la figure 12 montre une très forte activité promotrice du LTR issu de plasma SEP (c16) et une activité significativement beaucoup plus faible avec les séquences d'origine endogène non SEP.

### EXEMPLE 2: OBTENTION DU CLONE C15 CONTENANT LA REGION CODANT POUR UNE PARTIE DE L'ENVELOPPE DU RETROVIRUS MSRV-1

Une RT-PCR a été effectuée sur de l'ARN total extrait de virions concentrés par ultracentrifugation à partir d'un surnageant de culture de synoviocytes provenant d'un patient PR. La synthèse de cDNA a été réalisée avec une amorce oligo dT et la transcriptase inverse "Expand™ RT" de Boehringer selon les conditions préconisées par la société. Une PCR a été effectuée avec l'Expand™ Long Template PCR System (Boehringer) sous les conditions suivantes : 94°C 5 min puis 93°C 1 min, 60°C 1 min, 68°C 3 min pendant 40 cycles et 68°C pendant 8 min et avec un volume réactionnel final de 50 µl.
Amorces utilisées pour la PCR:
- amorce 5', identifiée par SEQ ID NO: 69
- amorce 3', identifiée par SEQ ID NO: 116

Une deuxième PCR dite "semi-nichée" a été réalisée avec une amorce 5' située à l'intérieur de la région déjà amplifiée. Cette deuxième PCR a été effectuée sous les mêmes conditions expérimentales que celles utilisées lors de la première PCR (sauf que 30 cycles ont été réalisés au lieu de 40), en utilisant 10 µl du produit d'amplification issu de la première PCR.
Amorces utilisées pour la PCR semi-nichée:
- amorce 5', identifiée par SEQ ID NO: 70
- amorce 3', identifiée par SEQ ID NO: 116
Les amorces SEQ ID NO: 69 et SEQ ID NO: 70 sont spécifiques de la région pol de MSRV-1. L'amorce SEQ ID NO: 116 est spécifique de la séquence FBd13 (aussi dénommé B13) et est localisée dans la région env conservée parmi les oncorétrovirus.

Un produit d'amplification de 1932 pb a été obtenu et cloné de la façon suivante :
l'ADN amplifié a été inséré dans un plasmide à l'aide du kit TA Cloning®. Les différentes étapes ont été réalisées conformément aux instructions du kit TA Cloning® (Invitrogen). A la fin de la procédure, les colonies blanches de bactéries recombinantes (white) ont été repiquées pour être cultivées et permettre l'extraction des plasmides incorporés selon la procédure dite de "miniprep". La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur SP6 présent sur le plasmide de clonage du TA cloning kit®. La réaction préalable au séquençage a ensuite été effectuée selon la méthode préconisée pour l'utilisation du kit de séquençage "PRISM™ Ready Reaction AmpliTaq® FS, DyeDeoxy™ Terminator" (Applied Biosystems, ref. 402119) et le séquençage automatique a été réalisé sur les appareils 373 A et 377 Applied Biosystems, selon les instructions du fabricant.

Le clone C15 obtenu, contient une région correspondant à la région de l'enveloppe de MSRV-1, de 1481 pb.

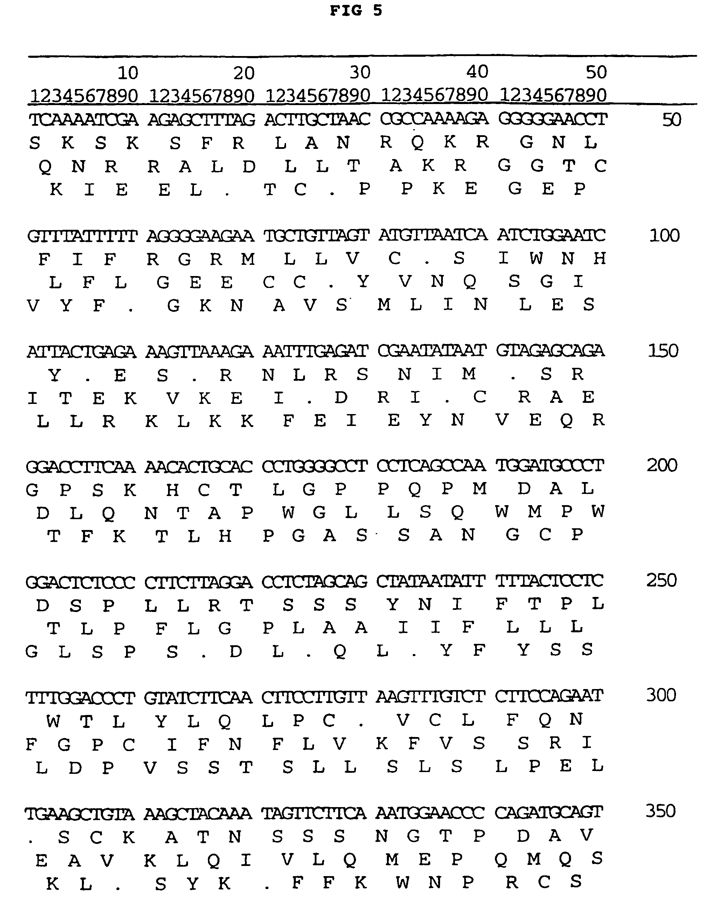

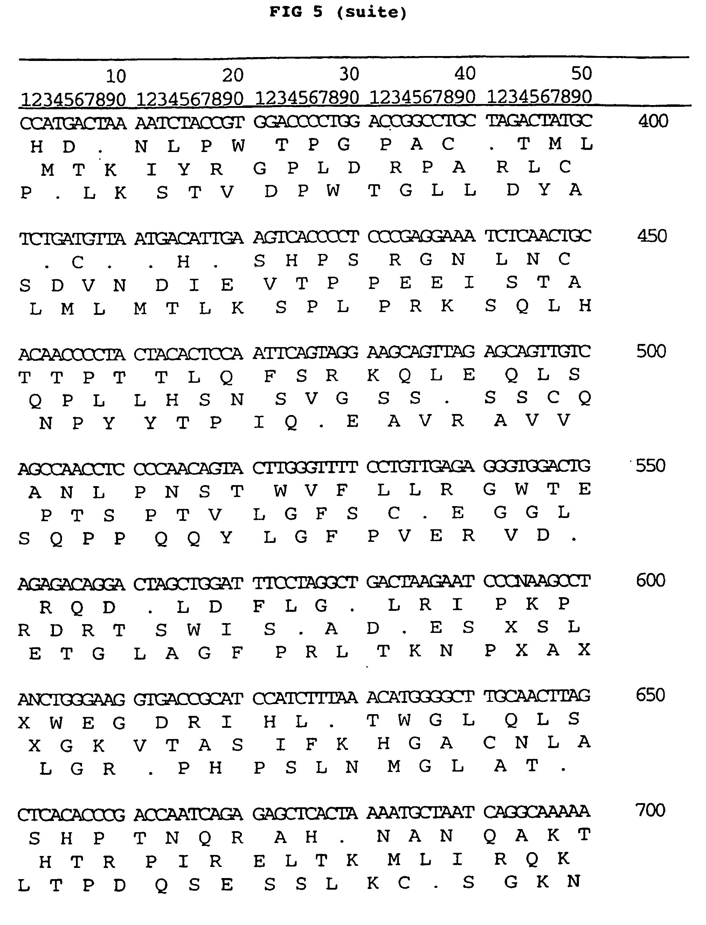

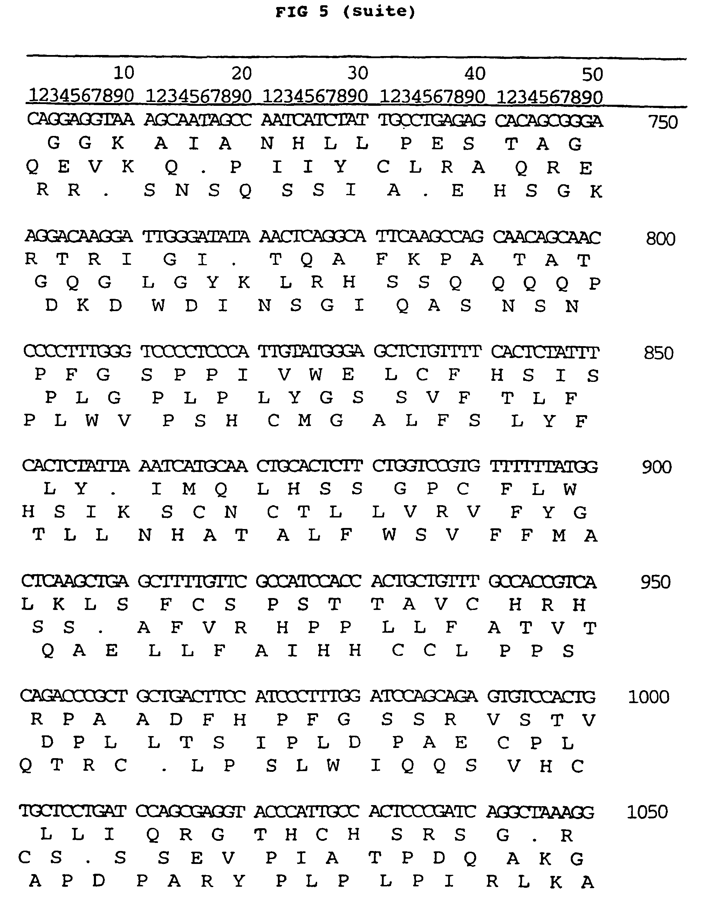

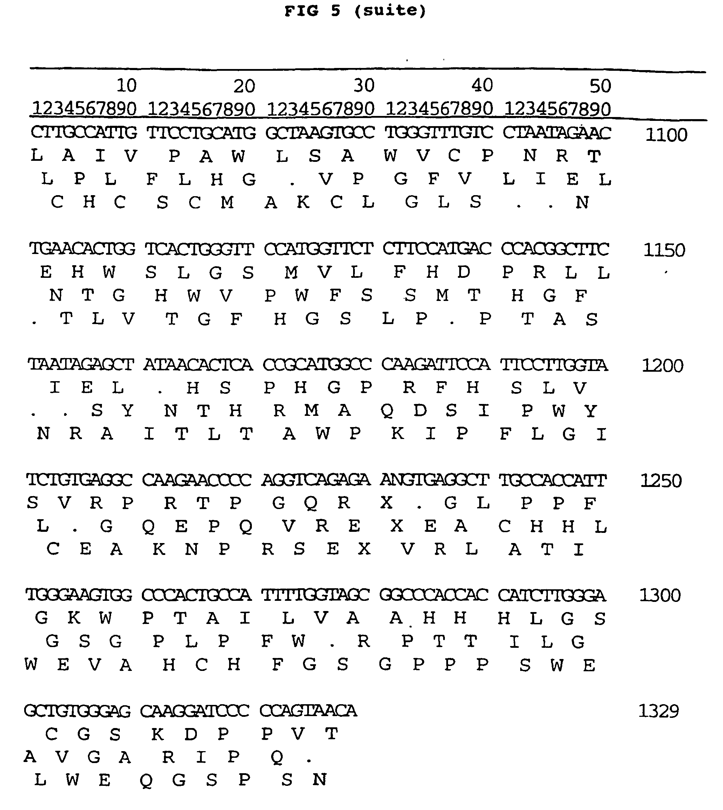

La région env du clone C15 est représentée par sa séquence nucléotidique (SEQ ID NO: 117) dans la figure 5. Les trois trames de lecture potentielles de ce clone sont présentées par leur séquence aminéoacide sous la séquence nucléotidique. La trame de lecture correspondant à une protéine env structurale MSRV-1 est identifiée par SEQ ID NO: 118.

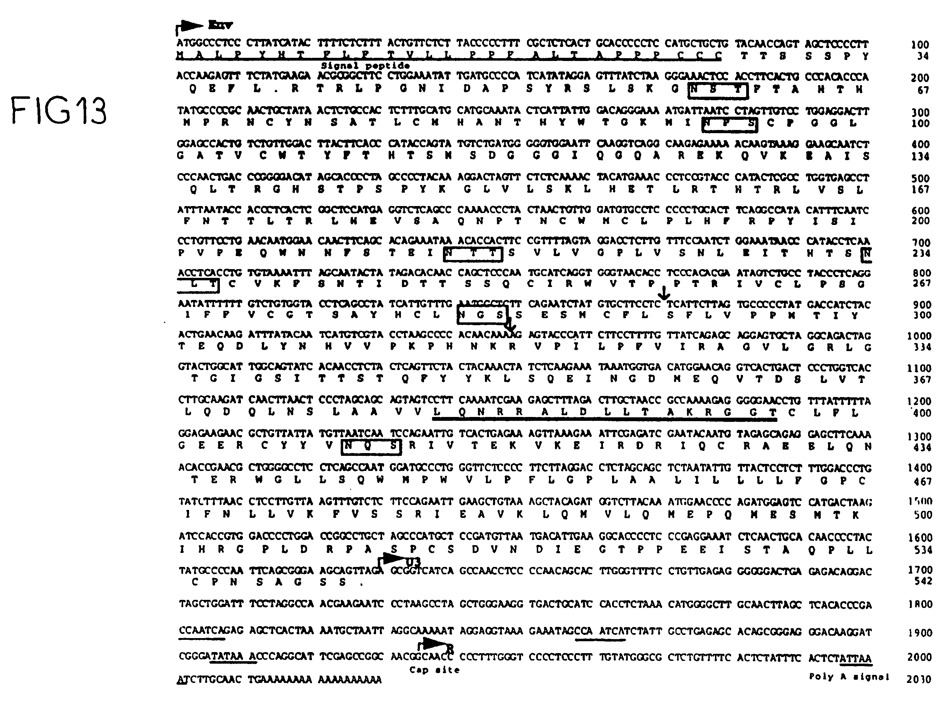

A partir des séquences définies provenant des clones c16 et C15, une construction plasmidique a pu être effectuée codant pour une enveloppe complète suivie du LTR3', comme présenté dans la figure 13 avec la trame de lecture correspondante.

### EXEMPLE 3: OBTENTION D'UN CLONE 5M6 CONTENANT LES SEQUENCES DE LA REGION 3' TERMINALE DE L'ENVELOPPE, SUIVIES DES SEQUENCES U3,R,U5 DE TYPE PROVIRAL MSRV-1.

Une PCR monodirectionnelle a été effectuée sur de l'ADN extrait de lymphocytes B immortalisés en culture d'un patient PR. La PCR a été effectuée avec l'Expand™ Long Template PCR System (Boehringer) sous les conditions suivantes : 94°C 3 min puis 93°C 1 min, 60°C 1 min, 68°C 3 min pendant 10 cycles , puis 93°C 1 min, 60°C 1 min avec 15 sec d'extension à chaque cycle, 68°C 3 min pendant 35 cycles et 68°C pendant 7 min et avec un volume réactionnel final de 50 µl.

L'amorce utilisée pour la PCR identifiée par SEQ ID NO: 119 est 5' TCA AAA TCG AAG AGC TTT AGA CTT GCT AAC CG 3' ;
L'amorces SEQ ID NO: 119 est spécifique de la région env du clone C15.

Un produit d'amplification de 1673 pb a été obtenu et cloné de la façon suivante :
l'ADN amplifié a été inséré dans un plasmide à l'aide du kit TA Cloning®. Les différentes étapes ont été réalisées conformément aux instructions du kit TA Cloning® (Invitrogen). A la fin de la procédure, les colonies blanches de bactéries recombinantes (white) ont été repiquées pour être cultivées et permettre l'extraction des plasmides incorporés selon la procédure dite de "miniprep". La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur T7 présent sur le plasmide de clonage du TA cloning kit®. La réaction préalable au séquençage a ensuite été effectuée selon la méthode préconisée pour l'utilisation du kit de séquençage "PRISM™ Ready Reaction AmpliTaq® FS, DyeDeoxy™ Terminator" (Applied Biosystems, ref. 402119) et le séquençage automatique a été réalisé sur les appareils 373 A et 377 Applied Biosystems, selon les instructions du fabricant.

Le clone 5M6 obtenu, contient une région correspondant à la région 3' de l'enveloppe de MSRV-1, de 492 pb suivi des régions U3, R et U5 (837 pb) de MSRV1.

Le clone 5M6 est représenté par sa séquence nucléotidique (SEQ ID NO: 120) dans la figure 5. Les trois trames de lecture potentielles de ce clone sont présentées par leur séquence aminéoacide sous la séquence nucléotidique. La trame de lecture correspondant à l'extrémité C-terminale de la protéine env MSRV-1 est identifiée par SEQ ID NO: 121.

### EXEMPLE 4: OBTENTION DU CLONE LB16 CONTENANT LA REGION CODANT L'INTEGRASE DU RETROVIRUS MSRV-1

Une RT-PCR a été effectuée sur de l'ARN total traité à la DNAseI et extrait à partir d'un plexus choroïde provenant d'un patient SEP. La synthèse de cDNA a été réalisée avec une amorce oligo dT et la transcriptase inverse "Expand™ RT" de Boehringer selon les conditions préconisées par la société. Un contrôle "no RT" a été effectué parallèlement sur le même matériel. Une PCR a été effectuée avec la Taq polymerase (Perkin Elmer) sous les conditions suivantes : 95°C 5 min puis 95°C 1 min, 55°C 1 min, 72°C 2 min pendant 35 cycles et 72°C pendant 8 min et avec un volume réactionnel final de 50 µl.
Amorces utilisées pour la PCR:
- amorce 5', identifiée par SEQ ID NO: 122
- amorce 3', identifiée par SEQ ID NO: 123

L'amorce SEQ ID NO: 122 est spécifique de la région pol de MSRV-1 et plus précisément similaire à la région intégrase décrite précédement. L'amorce SEQ ID NO 123 a été définie sur des séquences des clones obtenus lors d'essais préalables.

Un produit d'amplification d'environ 760 pb a été obtenu uniquement dans l'essai avec RT et a été cloné de la façon suivante :
l'ADN amplifié a été inséré dans un plasmide à l'aide du kit TA Cloning®. Les différentes étapes ont été réalisées conformément aux instructions du kit TA Cloning® (Invitrogen). A la fin de la procédure, les colonies blanches de bactéries recombinantes (white) ont été repiquées pour être cultivées et permettre l'extraction des plasmides incorporés selon la procédure dite de "miniprep". La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur T7 présent sur le plasmide de clonage du TA cloning kit®. La réaction préalable au séquençage a ensuite été effectuée selon la méthode préconisée pour l'utilisation du kit de séquençage "PRISM™ Ready Reaction AmpliTaq® FS, DyeDeoxy™ Terminator" (Applied Biosystems, ref. 402119) et le séquençage automatique a été réalisé sur les appareils 373 A et 377 Applied Biosystems, selon les instructions du fabricant.

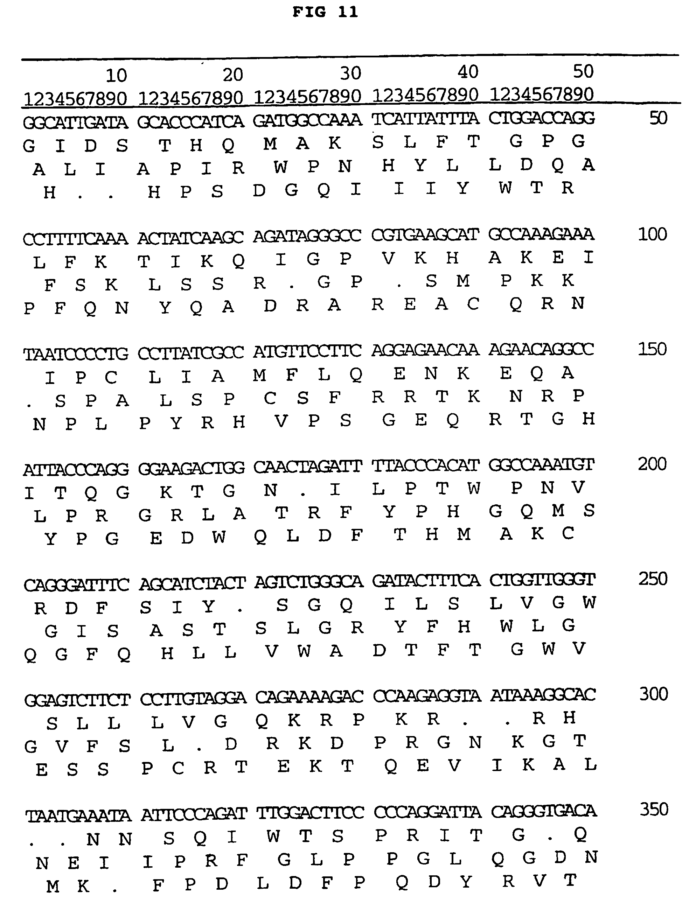

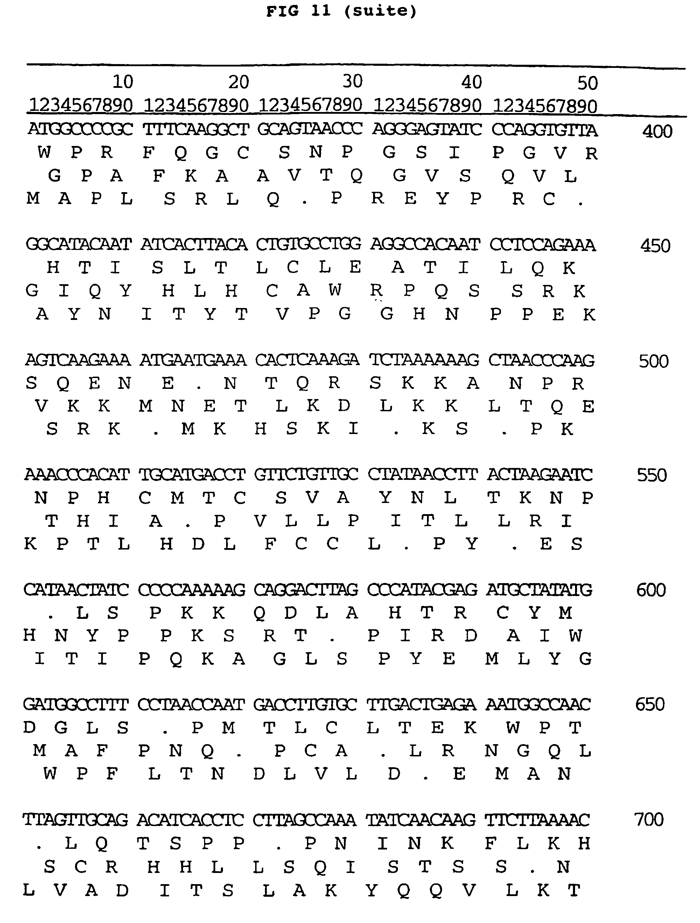

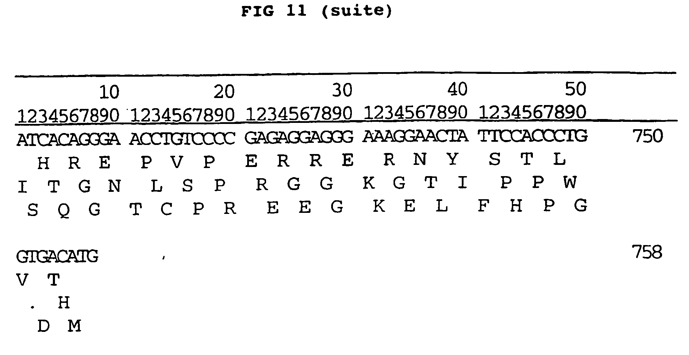

Le clone LB16 obtenu, contient les séquences correspondant à l'intégrase. La séquence nucléotidique de ce clone est identifiée par SEQ ID NO: 124 sur la figure 11, trois trames de lecture sont déterminées.

### EXEMPLE 5: OBTENTION D'UN CLONE 2, CL2, CONTENANT EN 3' UNE PARTIE HOMOLOGUE AU GENE POL, CORRESPONDANT AU GENE PROTEASE, ET AU GENE GAG (GM3) CORRESPONDANT A LA NUCLEOCAPSIDE, ET UNE NOUVELLE REGION 5'CODANTE, CORRESPONDANT AU GENE GAG PLUS SPECIFIQUEMENT LA MATRICE ET LA CAPSIDE de MSRV-1.

Une amplification par PCR a été effectuée sur de l'ARN total extrait à partir de 100 µl de plasma d'un patient atteint de SEP. Un témoin eau, traité sous les mêmes conditions, a été utilisé comme contrôle négatif. La synthèse de cDNA a été réalisée avec 300 pmole d'une amorce aléatoire (GIBCO-BRL, France) et la transcriptase inverse "Expand RT" (BOEHRINGER MANNHEIM, France) selon les conditions préconisées par la société. Une amplification par PCR (" polymerase chain reaction ") a été effectuée avec l'enzyme *Taq* polymerase (Perkin Elmer, France) en utilisant 10 µl de cDNA sous les conditions suivantes: 94°C 2 min, 55°C 1 min, 72°C 2 min puis 94°C 1 min, 55°C 1 min, 72°C 2 min pendant 30 cycles et 72°C pendant 7 min et avec un volume réactionnel final de 50 µl.
Amorces utilisées pour l'amplification par PCR:
- amorce 5', identifiée par SEQ ID NO: 126
- amorce 3', identifiée par SEQ ID NO: 127

Une deuxième amplification par PCR dite "semi-nichée" a été réalisée avec une amorce 5' située à l'intérieur de la région déjà amplifiée. Cette deuxième PCR a été effectuée sous les mêmes conditions expérimentales que celles utilisées lors de la première PCR, en utilisant 10 µl du produit d'amplification issu de la première PCR.
Amorces utilisées pour l'amplification par PCR semi-nichée:
- amorce 5', identifiée par SEQ ID NO: 128
- amorce 3', identifiée par SEQ ID NO: 129

Les amorces SEQ ID NO: et SEQ ID NO: sont spécifiques de la région pol, clone G+E+A, plus spécifiquement la région E: position nucleotidique n° 423 à n° 448. Les amorces utilisées dans la région 5' ont été définies sur des séquences de clones obtenus lors d'essais préalables.

Un produit d'amplification de 1511 pb a été obtenu à partir de l'ARN extrait du plasma de patient SEP. Le fragment correspondant n'a pas été observé pour le témoin eau. Ce produit d'amplification a été cloné de la façon suivante.

L'ADN amplifié a été inséré dans un plasmide à l'aide du kit TA Cloning™. Les 2 µl de solution d'ADN ont été mélangés avec 5 µl d'eau distillée stérile, 1 µl d'un tampon de ligation 10 fois concentré "10X LIGATION BUFFER", 2 µl de "pCR™ VECTOR" (25 ng/ml) et 1 µl de "T4 DNA LIGASE". Ce mélange a été incubé une nuit à 14°C. Les étapes suivantes ont été réalisées conformément au instructions du kit TA Cloning® (Invitrogen). Le mélange a été étalé après transformation de la ligation dans des bactéries *E. coli* INVαF'. A la fin de la procédure, les colonies blanches de bactéries recombinantes ont été repiquées pour être cultivées et permettre l'extraction des plasmides incorporés selon la procédure dite de "minipréparation d'ADN" (17). La préparation de plasmide de chaque colonie recombinante a été coupée par l'enzyme de restriction Eco RI et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur T7 présent sur le plasmide de clonage du TA cloning kit®. La réaction préalable au séquençage a ensuite été effectuée selon la méthode préconisée pour l'utilisation du kit de séquençage "PRISM™ Ready Reaction Amplitaq® FS, DyeDeoxy™ Terminator" (Applied Biosystems, ref. 402119) et le séquençage automatique a été réalisé sur les appareils 373 A et 377 Applied Biosystems, selon les instructions du fabricant.

Le clone obtenu, dénommé CL2, contient une région C-terminale similaire à la région 5' terminale des clones G+E+A de MSRV-1, qui permet de définir la région C-terminale du gène gag et une nouvelle région correspondante à la région N-terminale du gène gag MSRV-1.

CL2 permet de définir une région de 1511 pb présentant une phase ouverte de lecture dans la région N-terminale de 1077 pb codante pour 359 acides aminéés et une phase non-ouverte de lecture, de 454 pb, correspondant à la région C-terminale du gène gag MSRV-1.

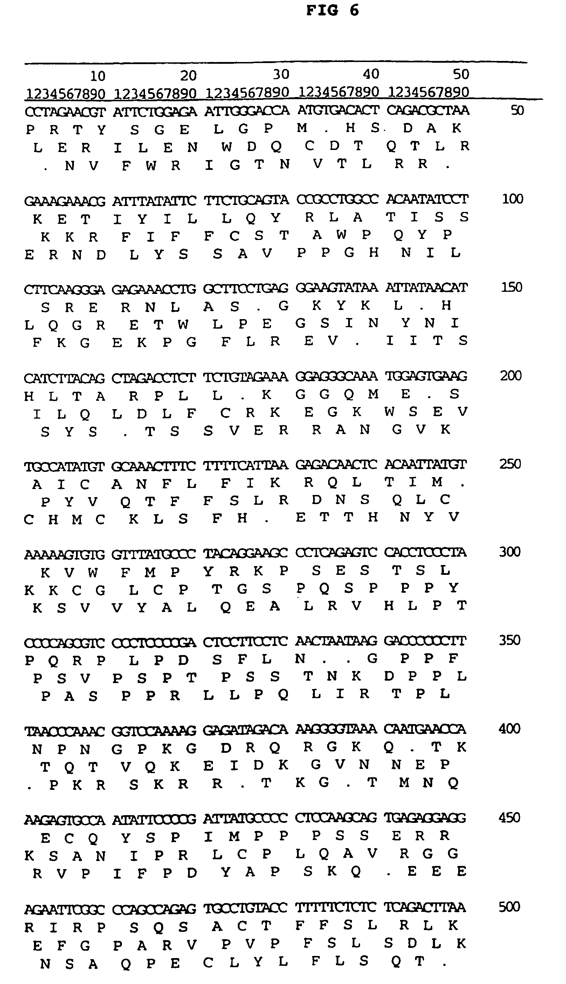

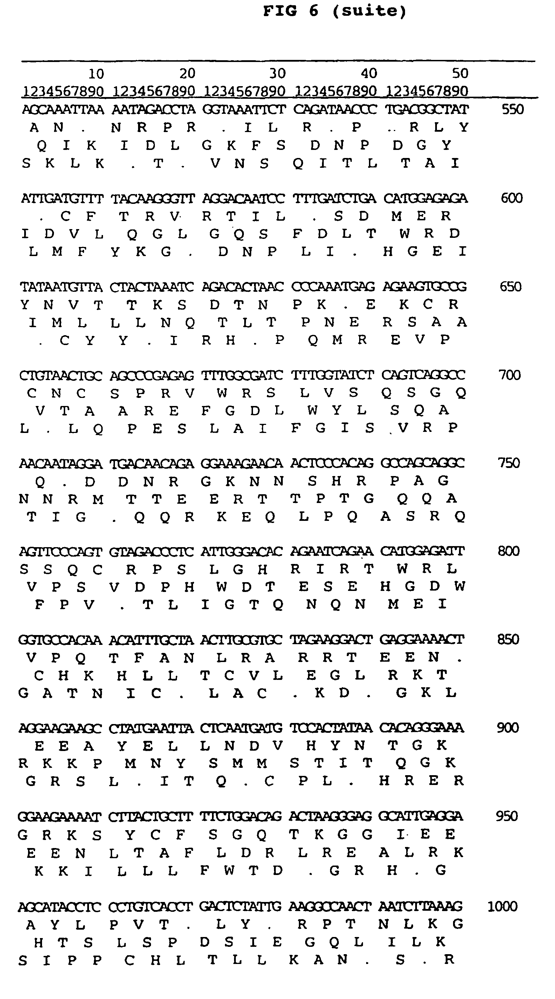

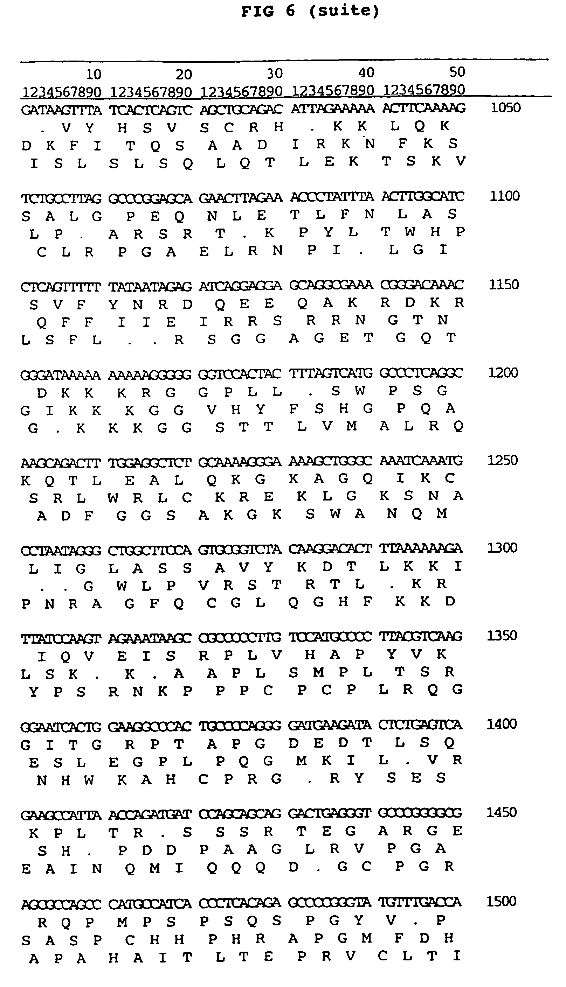

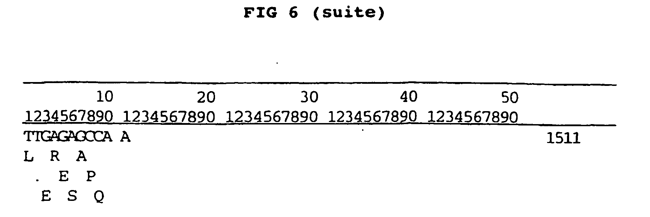

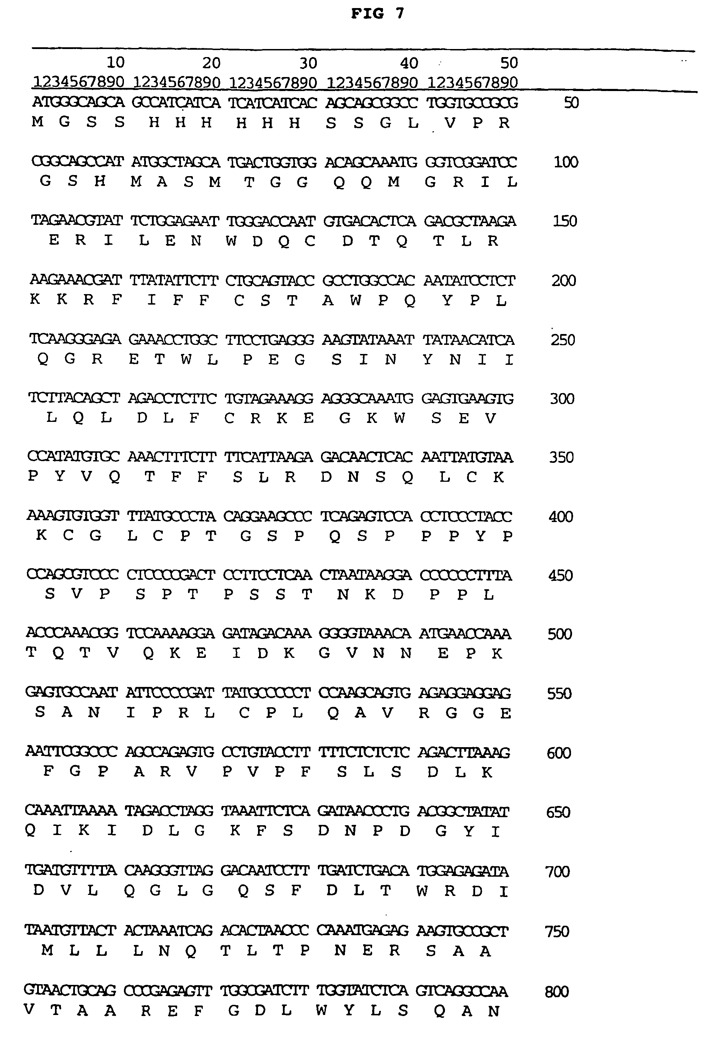

La séquence nucléotidique de CL2 est identifiée par SEQ ID NO: 130. Elle est représentée à la figure 6, avec les trames de lecture potentielles en aminéoacide.

Le fragment de 1077 pb de CL2 codant pour 359 acides aminés a été amplifié par PCR avec l'enzyme Pwo (5U/µl)(Boehringer Mannheim, France) en utilisant 1 µl de la minipréparation de l'ADN du clone 2 sous les conditions suivantes : 95°C 1 min, 60°C 1 min, 72°C 2 min pendant 25 cycles et avec un volume réactionnel final de 50 µl à l'aide des amorces:
- amorce 5' (*Bam* HI), identifié par SEQ ID NO: 132 et
- amorce 3' *(Hind* III), identifié par SEQ ID NO: 133 correspondant, respectivement, à la séquence nucléotidique du clone 2 en position -9 à 21 et 1066 à 1095.

Le fragment obtenu après PCR, a été linéarisé par *Bam* HI et *Hind*III et sous-cloné dans les vecteurs d'expression pET28C et pET21C (NOVAGEN) linéarisé par *Bam* HI et *Hind* III. Le séquençage de l'ADN du fragment de 1077 pb du clone 2 dans les deux vecteurs d'expression a été réalisé selon la méthode préconisée pour l'utilisation du kit de séquençage "PRISM™ Ready Reaction Amplitaq® FS, DyeDeoxy™ Terminator" (Applied Biosystems, ref. 402119) et le séquençage automatique a été réalisé sur les appareils 373 A et 377 Applied Biosystems, selon les instructions du fabricant.

L'expression de la séquence nucléotidique du fragment de 1077 pb du clone 2 par les vecteurs d'expression pET28C et pET21C sont identifiées par respectivement SEQ ID NO: 135 et SEQ ID NO: 137.

### EXEMPLE 6: EXPRESSION DU CLONE 2 CHEZ ESCHERICHIA COLI

Les constructions pET28c-clone 2 (1077 pb) et pET21C-clone 2 (1077 pb) synthétisent, dans la souche bactérienne BL21 (DE3), une protéine en fusion N- et C-terminale pour le vecteur pET28C et C-Terminale pour le vecteur pET21C avec 6 Histidines, de masse moléculaire apparente d'environ 45 kDa, mise en évidence par électrophorèse sur gel de polyacrylamide SDS-PAGE (SDS = Dodecyl Sulfate de Sodium) (Laemmli, 1970 (1)). La réactivité de la protéine a été mise en évidence vis à vis d'un anticorps monoclonal anti-Histidine (DIANOVA) par la technique de Western blot (Towbin et al., 1979 (2)).

Les protéines recombinantes pET28c-clone 2 (1077 pb) et pET21C-clone 2 (1077 pb) ont été visualisées en SDS-PAGE dans la fraction insoluble après digestion enzymatique des extraits bactériens avec 50 µl de lysozyme (10 mg/ml) et lyse par ultrasons.

Les propriétés antigéniques des antigènes recombinants pET28C-clone 2 (1077 pb) et pET21C-clone 2 (1077 pb) ont été testées par Westen Blot () après solubilisation du culot bactérien avec 2% SDS et 50 mM β-mercaptoéthanol. Après incubation avec les sérums de patients atteints de sclérose en plaques, les sérums des témoins neurologiques et les sérums de témoins de centre de transfusion sanguine (CTS), les immunocomplexes ont été detectés à l'aide d'un sérum de chèvre anti-IgG et anti IgM humaines, couplé à la phosphatase alcaline.

Les résultats sont présentés dans le tableau ci-après.

**TABLEAU**

| Réactivite de sérums atteints de sclérose en plaques et témoins avec la protéine recombinante MSRV-1 gag clone 2 (1077 pb) = pET21C-clone 2 (1077 pb) et pET28C-clone 2 (1077 pb)^{a} | | |
|---|---|---|
| MALADIE | NOMBRE D'INDIVIDUS TESTÉS | NOMBRE D'INDIVIDUS POSITIFS |
| SEP | 15 | 6 2 (+++), 2 (++), 2 (+) |
| TEMOINS NEUROLOGIQUES | 2 | 1(+++) |
| TEMOINS SAINS (CTS) | 22 | 1(+/-) |

| | | |
|---|---|---|
| (a) Les bandelettes contenant 1,5 µg d'antigène recombinant pET-gag clone 2 (1077 pb) présentent une réactivité contre de sérums dilués au 1/100. L'interprétation de Western Blot est basée sur la présence ou absence d'une bande pET-gag clone 2 (1077 pb) spécifique sur les bandelettes. Des contrôles positifs et négatifs sont inclus dans chaque expérience. | | |

Ces résultats montrent que, dans les conditions techniques utilisées, environ 40% des sérums humains atteints de sclérose en plaques testés réagissent avec les protéines recombinantes pET28C-clone 2 (1077 pb) et pET21C-clone 2 (1077 pb). Une réactivité a été observée sur un témoin neurologique et il est intéressant de noter que les ARN extraits à partir de ce sérum, après l'étape de transcriptase inverse, sont aussi amplifiés par PCR dans la région pol. Ceci suggère que des personnes n'ayant pas déclaré une SEP peuvent également héberger et exprimer ce virus. Par contre, un témoin (donneur CTS) apparemment sain, possède des anticorps anti-gag (clone 2, 1077 pb). Ce qui est compatible avec un immunité acquise contre MSRV-1 en dehors d'une maladie autoimmune associée déclarée.

### EXEMPLE 7: OBTENTION D'UN CLONE LB13 CONTENANT EN 3' UNE PARTIE HOMOLOGUE AU CLONE 2 CORRESPONDANT AU GENE GAG ET EN 5' UNE PARTIE HOMOLOGUE AU CLONE 5M6 CORRESPONDANT À LA RÉGION LTR U5.

Une RT-PCR (" reverse-transcriptase-polymerase chain reaction ") a été effectuée à partir de l'ARN total extrait de virions, provenant de surnageants de cellules lymphocytaires B des patients atteints de sclérose en plaques, concentrés par ultracentrifugations. La synthèse de cDNA a été realisée avec une amorce spécifique SEQ N° XXX et la transcriptase inverse " Expand™ RT " de BOEHRINGER MANNHEIM selon les conditions preconisées par la société.
Amorce utilisée pour la synthèse du cDNA, identifiée par SEQ ID NO: 138:

Une amplification par PCR a été réalisée avec la *Taq* polymérase (Perkin Elmer, France) sous les conditions suivantes: 94°C 1 min, 55 °C 1 min, 72 °C 2 min pendant 35 cycles et 72°C pendant 7 min et avec un volume réactionnel final de 100 µl.
Amorces utilisées pour l'amplification par PCR:
- amorce 5', identifiée par SEQ ID NO: 139
- amorce 3', identifiée par SEQ ID NO: 138

Une deuxième amplification par PCR dite " semi-nichée " a été réalisée avec une amorce 3' située à l'intérieur de la région déjà amplifiée. Cette deuxième amplification a été effectuée sous les mêmes contitions expérimentales que celles utilisées lors de la première amplification, en utilisant 10 µl du produit d'amplification issu de la première PCR.
Amorces utilisées por l'amplification par PCR " semi-nichée ":
- amorce 5', identifiée par SEQ ID NO: 139
- amorce 3', identifiée par SEQ ID NO: 140

Les amorces SEQ ID NO: 138 et SEQ ID NO: 140 sont spécifiques de la région gag, clone 2 position nucléotidique n° 373-397 et n° 433-456. Les amorces utilisées dans la région 5' ont été définies sur des séquences des clones obtenus lors d'essais préalables.

Un produit d'amplification de 764 pb a été obtenu et cloné de la façon suivante:
L'ADN amplifié a été inséré dans un plasmide à l'aide du kit TA Cloning™. Les 2 µl de solution d'ADN ont été mélangés avec 5 µl d'eau distillée stérile, 1 µl d'un tampon de ligation 10 fois concentré "10X LIGATION BUFFER", 2 µl de "pCR™ VECTOR" (25 ng/ml) et 1 µl de "T4 DNA LIGASE". Ce mélange a été incubé une nuit à 14°C. Les étapes suivantes ont été réalisées conformément au instructions du kit TA Cloning® (Invitrogen). Le mélange a été étalé après transformation de la ligation dans des bactéries *E. coli* INVαF'. A la fin de la procédure, les colonies blanches de bactéries recombinantes ont été repiquées pour être cultivées et permettre l'extraction des plasmides incorporés selon la procédure dite de "minipréparation d'ADN" (17). La préparation de plasmide de chaque colonie recombinante a été coupée par l'enzyme de restriction Eco RI et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur T7 présent sur le plasmide de clonage du TA cloning kit®. La réaction préalable au séquençage a ensuite été effectuée selon la méthode préconisée pour l'utilisation du kit de séquençage "PRISM™ Ready Reaction Amplitaq® FS, DyeDeoxy™ Terminator" (Applied Biosystems, ref. 402119) et le séquençage automatique a été réalisé sur les appareils 373 A et 377 Applied Biosystems, selon les instructions du fabricant.

Le clone LB13 obtenu contient une région N-terminale de gène gag MSRV-1 homologue au clone 2 et un LTR correspondant à une partie de la région U5. Entre la région U5 et gag un site de fixation pour les ARN de transfert, le PBS " primer binding site " a été identifié.

La séquence nucléotidique du fragment de 764 pb du clone LB13 dans le plasmide "pCR™ vector" est représentée dans l'identificateur SEQ ID NO: 141.

Le site de fixation pour les ARN de transfert, présentant une séquence du type PBS tryptophane, a été identifié en position nucléotidique n°342-359 du clone LB13.

Comme ce même PBS a été retrouvé dans les copies endogènes homologues à MSRV1, la famille endogène ainsi définie est appelée dorénavant HERV W, selon la nomenclature proposée pour les familles de rétrovirus endogènes (W=Tryptophane).

Une ORF courte d'environ 65 acides aminés a été retrouvée dans la région U5 du LTR 5' du clone LB13.

Séquence de l'ORF :

La séquence nucléotidique correspondante débutant par un codon ATG est susceptible d'être exprimée dans un ADN sous génomique à partir d'un LTR proviral (U3RU5).

Un autre clone, dénommé LA15 a été obtenu sur l'ARN total extrait de virions concentrés par ultracentrifugation à partir d'un surnageant de culture de synoviocytes provenant d'un patient atteint de polyarthrite rhumatoide. La stratégie d'amplification et clonage du clone LA15 est exactement la même qui a été utilisée pour le clone LB13.

La séquence nucléotidique du clone LA15 qui est représentée dans l'identificateur SEQ ID NO: 142, est très similaire au clone LB13. Ceci suggère que le rétrovirus MSVR-1 détecté dans la sclérose en plaque présente des séquences similaires à celles rencontrées dans la polyarthrite rhumatoïde.

### EXEMPLE 8: RECONSTRUCTION D'UNE REGION RU5-GAG A PARTIR DES CLONES LB15, LB13, CL2 ET CL17

Les clones CL2 et LB13 ont déjà été décrits dans les exemples précédents. Le clone LB15 a été obtenu en utilisant la séquence R du LTR du clone cl6 pour définir une amorce en 5' et les amorces anti-sens utilisées sont les mêmes que pour le clone LB13. Le clone CL17 a été obtenu par nested RT-PCR en utilisant les amorces suivantes :

Le clone LB15 a été obtenu à partir de virions obtenus par culture de cellules de SEP. Le clone LB17 a été obtenu à partir de culture de plasma de patient SEP.

Ces clones chevauchants ont permis de reconstruire une séquence RU5-gag avec une ORF potentielle dans le gène gag, telles que présentées à la figure 14.

### EXEMPLE 9: OBTENTION D'UN CLONE 87-23

La région correspondant à l'intégrase a été amplifiée et clonée à partir de plasma de SEP en utilisant une semi-nested RT PCR avec les amorces suivantes situées dans les régions pol et env de MSRV1.
Dans la région pol : Dans la région env,

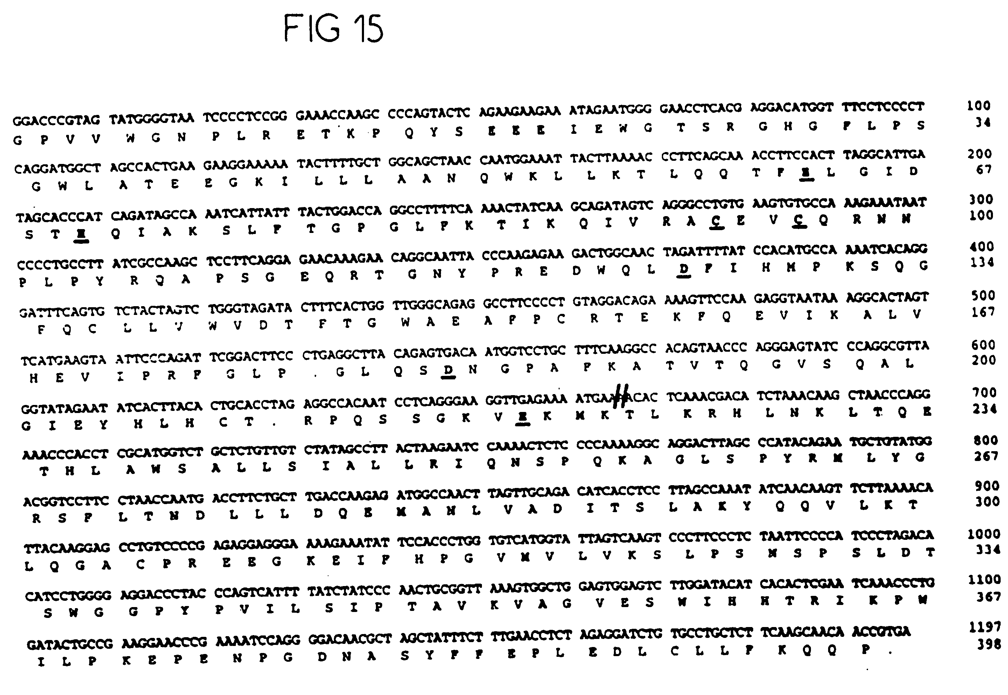

Le clone amplifié comporte 774 pb dans la région pol/RT, toute la région intégrase (1197 pb) et le début de la région env (480 pb). La séquence nucléotidique correspondant à la région intégrase et la traduction en acides aminés de l'ORF potentiel sont présentés à la figure 15.

### EXEMPLE 10: CONFIRMATION DE LA PRESENCE D'ARN CONTENANT DES SEQUENCES ENV APPARENTEES A ERV9 DANS LES PARTICULES RETROVIRALES ASSOCIEES AU GENOME MSRV1 :

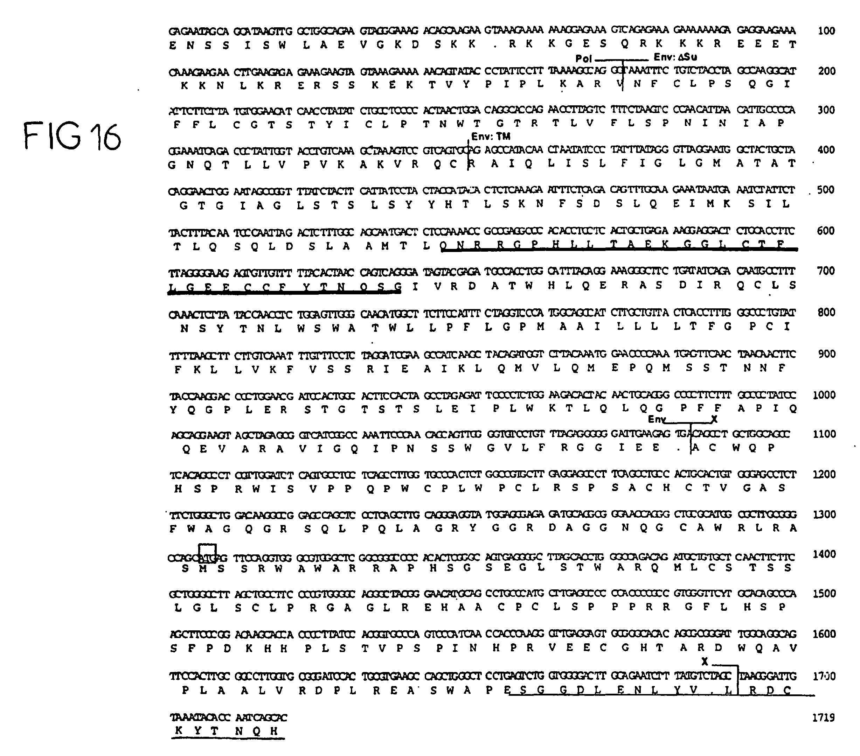

Des séquences apparentées à ERV9 ont été trouvées en proportion minoritaire dans les préparations du virion provenant de SEP par rapport aux séquences MSRV1. L'existence de phénomènes de co-encapsidation de séquences endogènes phylogéniquement proches dans des particules rétrovirales produites par une souche réplicative a été décrite. De manière surprenante une région d'ARN comprenant une ORF commençant dans la partie 3' d'env et se continuant potentiellement dans le LTR3' a été retrouvée dans différents échantillons de SEP. Afin de préciser l'existence d'une ORF des essais de transcription-traduction ont été réalisés et ont permis de montrer la réalité d'une ORF env contenant toute la partie transmembranaire (TM) et se terminant au début du LTR putatif. Cependant une trame additionnelle (ORFX) fait suite et se poursuit dans le LTR3'. Les deux produits d'expression ont été visualisés et leurs ORFs respectives ont été sous clonées. La figure 16 représente les séquences nucléotidique et peptidique du clone B13 déjà décrit en précisant les ORFs dans la région env tronquée et dans le LTR putatif. La présence de tels ARN peut être à l'origine de recombinaisons avec la souche replicative et par conséquent générer des souches de pathogénécité modifiée.

### BIBLIOGRAPHIE

(1) Laemmli U. K. Cleavage of structural proteins during the assembly of the head of bacteriophage T4. Nature. (1970). **227**: 680-685.
(2) Towbin H., Staehelin T. & Gordon J. Electrophoretic transfer of proteins from polyacrylamide gels to nitrocellulose sheets: procedure and some applications. Proc. Natl. Acad. Sci. USA. (1979). **76**: 4350-4354.

## Revendications

1. Matériel nucléique, à l'état isolé ou purifié, comprenant une séquence nucléotidique choisie dans le groupe qui consiste en (i) les séquences SEQ ID NO: 114, SEQ ID NO: 117, SEQ ID NO: 120 ; (ii) les séquences complémentaires des séquences (i) ; et (iii) les séquences équivalentes aux séquences (i) ou (ii) présentant pour toute suite de 100 monomères contigus, au moins 50 % d'homologie avec respectivement les séquences (i) ou (ii), ledit matériel nucléique étant différent de la séquence HSAC64 du clone humain RG083M05 accessible sur la base de donnée EMBL, sous le numéro AC000064.

2. Matériel nucléique selon la revendication 1, **caractérisé en ce qu'**il comprend une séquence nucléotidique choisie dans le groupe (iii) des séquences équivalentes aux séquences (i) ou (ii) présentant pour toute suite de 100 monomères contigus, au moins 70% d'homologie avec respectivement les séquences (i) ou (ii).

3. Matériel nucléique, à l'état isolé ou purifié, codant pour un polypeptide présentant, pour toute suite contiguë d'au moins 30 acides aminés, au moins 50 % d'homologie, avec une séquence peptidique choisie dans le groupe qui consiste en SEQ ID N°115, SEQ ID NO: 118, SEQ ID NO: 121, ledit matériel nucléique étant différent de la séquence HSAC64 du clone humain RG083M05 accessible sur la base de donnée EMBL, sous le numéro AC000064.

4. Matériel nucléique selon la revendication 3, **caractérisé en ce qu'**il code pour un polypeptide présentant, pour toute suite contiguë d'au moins 30 acides aminés, au moins 70 % d'homologie, avec une séquence peptidique choisie dans le groupe qui consiste en SEQ ID N°115, SEQ ID NO: 118, SEQ ID NO: 121.

5. Matériel nucléique rétroviral, dont le gène *env* comprend une séquence nucléotidique choisie dans le groupe qui consiste en SEQ ID NO : 114, SEQ ID NO: 117, et SEQ ID NO : 120 ; et leurs séquences complémentaires.

6. Matériel nucléique rétroviral, dont le gène *env* comprend une séquence nucléotidique qui commence au nucléotide 1 de SEQ ID NO: 117 et finit au nucléotide 233 de SEQ ID NO: 114.

7. Matériel nucléique rétroviral, dont le gène env code pour un polypeptide présentant, pour toute suite contiguë d'au moins 30 acides aminés, au moins 50 % d'homologie, avec la séquence SEQ ID NO:°118.

8. Matériel nucléique selon la revendication 7, **caractérisé en ce que** son gène env code pour un polypeptide présentant, pour toute suite contiguë d'au moins 30 acides aminés, au moins 70 % d'homologie, avec la séquence SEQ ID NO:°118

9. Matériel nucléique rétroviral dont la région U3R du LTR 3' comprend une séquence nucléotidique qui se termine au nucléotide 617 de SEQ ID NO: 114.

10. Matériel nucléique rétroviral dont la région RU5 du LTR 5' comprend une séquence nucléotidique qui commence au nucléotide 755 de SEQ ID NO: 120.

11. Matériel nucléique rétroviral comprenant une séquence qui commence au nucléotide 755 de SEQ ID NO: 120 et qui se termine au nucléotide 617 de SEQ ID NO: 114.

12. Matériel nucléique rétroviral selon l'une quelconque des revendications précédentes, **caractérisé en ce qu'**il est associé à au moins une maladie auto-immune choisie parmi la sclérose en plaques et la polyarthrite rhumatoïde.

13. Fragment nucléotidique comprenant une séquence nucléotidique choisie dans le groupe qui consiste en (i) les séquences SEQ ID NO: 114, SEQ ID NO: 117, SEQ ID NO: 120 ; (ii) les séquences complémentaires des séquences (i) ; et (iii) les séquences équivalentes aux séquences (i) ou (ii) présentant pour toute suite de 100 monomères contigus, au moins 50 % d'homologie avec respectivement les séquences (i) ou (ii), ledit fragment nucléotidique étant différent de la séquence HSAC64 du clone humain RG083M05 accessible sur la base de donnée EMBL, sous le numéro AC000064.

14. Fragment nucléotidique selon la revendication 13 **caractérisé en ce qu'**il comprend une séquence nucléotidique choisie dans le groupe (iii) des séquences équivalentes aux séquences (i) ou (ii) présentant pour toute suite de 100 monomères contigus, au moins 70% d'homologie avec respectivement les séquences (i) ou (ii).

15. Fragment nucléotidique selon la revendication 13, consistant en une séquence nucléotidique choisie dans le groupe qui consiste en (i) les séquences SEQ ID NO: 114, SEQ ID NO: 117, SEQ ID NO: 120 ; (ii) les séquences complémentaires des séquences (i) ; et (iii) les séquences équivalentes aux séquences (i) ou (ii) présentant pour toute suite de 100 monomères contigus, au moins 50 % d'homologie avec respectivement les séquences (i) ou (ii).

16. Fragment nucléotidique selon la revendication 15, **caractérisé en qu'**il consiste en une séquence nucléotidique choisie dans le groupe (iii) des séquences équivalentes aux séquences (i) ou (ii) présentant pour toute suite de 100 monomères contigus, au moins 70% d'homologie avec respectivement les séquences (i) ou (ii) .

17. Fragment nucléotidique comprenant une séquence nucléotidique codant pour un polypeptide présentant, pour toute suite contiguë d'au moins 30 acides aminés, au moins 50 % d'homologie, avec une séquence peptidique choisie dans le groupe qui consiste en SEQ ID N°115, SEQ ID NO: 118, SEQ ID NO: 121, ledit fragment nucléotidique étant différent de la séquence HSAC64 du clone humain RG083M05 accessible sur la base de donnée EMBL, sous le numéro AC000064.

18. Fragment nucléotidique selon la revendication 17, **caractérisé en ce qu'**il code pour un polypeptide présentant, pour toute suite contiguë d'au moins 30 acides aminés, au moins 70 % d'homologie, avec une séquence peptidique choisie dans le groupe qui consiste en SEQ ID N°115, SEQ ID NO: 118, SEQ ID NO: 121.

19. Fragment nucléotidique selon la revendication 17, consistant en une séquence nucléotidique codant pour un polypeptide présentant, pour toute suite contiguë d'au moins 30 acides aminés, au moins 50 % d'homologie, avec une séquence peptidique choisie dans le groupe qui consiste en SEQ ID N°115, SEQ ID NO: 118 et SEQ ID NO : 121.

20. Fragment nucléotidique selon la revendication 19, **caractérisé en ce qu'**il consiste en une séquence nucléotidique codant pour un polypeptide présentant, pour toute suite contiguë d'au moins 30 acides aminés, au moins 70 % d'homologie, avec une séquence peptidique choisie dans le groupe qui consiste en SEQ ID N°115, SEQ ID NO: 118, SEQ ID NO: 121.

21. Sonde nucléique pour la détection d'un rétrovirus associé à la sclérose en plaques et/ou la polyarthrite rhumatoïde, **caractérisée en ce qu'**elle est susceptible de s'hybrider spécifiquement sur tout fragment selon l'une quelconque des revendications 13 à 20, appartenant au génome dudit rétrovirus et **en ce qu'**elle possède de 10 à 100 nucléotides.

22. Sonde selon la revendication 21, **caractérisée en ce qu'**elle possède de 10 à 30 nucléotides.

23. Amorce pour l'amplification par polymérisation d'un ARN ou d'un ADN d'un rétrovirus associé à la sclérose en plaques et/ou la polyarthrite rhumatoïde, **caractérisée en ce qu'**elle comprend une séquence nucléotidique identique à au moins une partie de la séquence nucléotidique d'un fragment selon l'une quelconque des revendications 13 à 20, ledit fragment nucléotidique appartenant au gène *env* dudit rétrovirus, et **en ce que** ladite partie de la séquence nucléotidique comprend au moins 6 monomères .

24. *Amorce* selon la revendication *23*, **caractérisée en ce qu'**elle comprend de 10 à 30 monomères.

25. Amorce pour l'amplification par polymérisation d'un ARN ou d'un ADN d'un rétrovirus associé à la sclérose en plaques et/ou la polyarthrite rhumatoïde, **caractérisée en ce qu'**elle comprend une séquence nucléotidique présentant pour toute suite de 10 monomères contigus, au moins 50 % d'homologie avec un fragment selon l'une quelconque des revendications *13 à 20*, ledit fragment nucléotidique appartenant au gène *env* dudit rétrovirus, et **en ce que** ladite séquence nucléotidique comprend de 10 à 30 monomères.

26. *Amorce selon la revendication 25,* ***caractérisée* en ce qu'**elle comprend une séquence nucléotidique présentant pour toute suite de 10 monomères contigus, au moins 70 % d'homologie avec ledit fragment.

27. Amorce selon la revendication 24, **caractérisée en ce que** sa séquence nucléotidique est choisie parmi SEQ ID NO: 116 et SEQ ID NO: 119.

28. Vecteur de réplication et/ou d'expression, comprenant un fragment nucléotidique selon l'une quelconque des revendications 13 à 20.

29. Peptide codé par tout cadre de lecture ouvert appartenant à un fragment nucléotidique selon l'une quelconque des revendications 13 à 20.

30. Peptide comprenant une séquence peptidique identique à une séquence choisie parmi SEQ ID N°115, SEQ ID NO: 118 et SEQ ID NO: 121.

31. Peptide comprenant une séquence peptidique qui pour toute suite contiguë d'au moins 30 acides aminés présente au moins 50 % d'homologie avec une séquence choisie parmi SEQ ID N°115, SEQ ID NO: 118 et SEQ ID NO: 121.

32. Peptide selon la revendication 31, **caractérisé en ce qu'**il comprend une séquence peptidique qui pour toute suite contiguë d'au moins 30 acides aminés présente au moins 70 % d'homologie avec une séquence choisie parmi SEQ ID N°115, SEQ ID NO: 118 et SEQ ID NO: 121.

33. Composition diagnostique, prophylactique, ou thérapeutique, comprenant un fragment nucléotidique selon l'une quelconque des revendications 13 à 20.

34. Procédé pour détecter un rétrovirus associé à la sclérose en plaques et/ou à la polyarthrite rhumatoïde, dans un échantillon biologique, **caractérisé en ce que** l'on met en contact un ARN et/ou un ADN présumé appartenir ou provenant dudit rétrovirus, ou leur ARN et/ou ADN complémentaire, avec une composition comprenant un fragment nucléotidique selon l'une quelconque des revendications 13 à 20.

## Patentansprüche

1. Nukleinsäuren in isolierter oder aufgereinigter Form, die eine Nukleotidsequenz aus der Gruppe (i) der Sequenzen SEQ ID NO: 114, SEQ ID NO: 117, SEQ ID NO: 120; (ii) der zu den Sequenzen (i) komplementären Sequenzen; sowie (iii) der zu den Sequenzen (i) oder (ii) äquivalenten Sequenzen, die über eine beliebige Reihe von 100 aufeinanderfolgenden Monomeren mindestens 50% Homologie mit den Sequenzen (i) bzw. (ii) aufweisen, umfassen, wobei sich die Nukleinsäuren von der Sequenz HSAC64 des menschlichen Klons RG083M05, der unter der Nummer AC000064 bei der EMBL-Datenbank verfügbar ist, unterscheiden.

2. Nukleinsäuren nach Anspruch 1, **dadurch gekennzeichnet, daß** sie eine Nukleotidsequenz aus der Gruppe (iii) der zu den Sequenzen (i) oder (ii) äquivalenten Sequenzen, die über eine beliebige Reihe von 100 aufeinanderfolgenden Monomeren mindestens 70% Homologie mit den Sequenzen (i) bzw. (ii) aufweisen, umfassen.

3. Nukleinsäuren im isolierten oder aufgereinigten Zustand, die für ein Polypeptid codieren, das über eine beliebige aufeinanderfolgende Reihe von mindestens 30 Aminosäuren mindestens 50% Homologie mit einer Peptidsequenz aus der Gruppe SEQ ID NO: 115, SEQ ID NO: 118, SEQ ID NO: 121 aufweist, wobei sich die Nukleinsäuren von der Sequenz HSAC64 des menschlichen Klons RG083M05, der unter der Nummer AC000064 bei der EMBL-Datenbank verfügbar ist, unterscheiden.

4. Nukleinsäuren nach Anspruch 3, **dadurch gekennzeichnet, daß** sie für ein Polypeptid codieren, das über eine beliebige aufeinanderfolgende Reihe von mindestens 30 Aminosäuren mindestens 70% Homologie mit einer Peptidsequenz aus der Gruppe SEQ ID NO: 115, SEQ ID NO: 118, SEQ ID NO: 121 aufweist.

5. Retrovirale Nukleinsäuren, bei denen das env-Gen eine Nukleotidsequenz aus der Gruppe SEQ ID NO: 114, SEQ ID NO: 117 und SEQ ID NO: 120 sowie ihre komplimentären Sequenzen umfaßt.

6. Retrovirale Nukleinsäuren, bei denen das env-Gen eine Nukleotidsequenz, die bei Nukleotid 1 der SEQ ID NO: 117 beginnt und bei Nukleotid 233 der SEQ ID NO: 114 aufhört, umfaßt.

7. Retrovirale Nukleinsäuren, bei denen das env-Gen für ein Polypeptid codiert, das über eine beliebige aufeinanderfolgende Reihe von mindestens 30 Aminosäuren mindestens 50% Homologie mit der SEQ ID NO: 118 aufweist.

8. Nukleinsäuren nach Anspruch 7, **dadurch gekennzeichnet, daß** ihr env-Gen für ein Polypeptid codiert, das über eine beliebige aufeinanderfolgende Reihe von mindestens 30 Aminosäuren mindestens 70% Homologie mit der SEQ ID NO: 118 aufweist.

9. Retrovirale Nukleinsäuren, deren U3R-Region des 3'-LTR eine Nukleotidsequenz, die bei Nukleotid 617 von SEQ ID NO: 114 aufhört, umfaßt.

10. Retrovirale Nukleinsäuren, deren RU5-Region des 5'-LTR eine Nukleotidsequenz, die bei Nukleotid 755 von SEQ ID NO: 120 beginnt, umfaßt.

11. Retrovirale Nukleinsäuren, die eine Sequenz, die bei Nukleotid 755 von SEQ ID NO: 120 beginnt und bei Nukleotid 617 von SEQ ID NO: 114 aufhört, umfassen.

12. Retrovirale Nukleinsäuren nach einem der vorhergehenden Ansprüche, **dadurch gekennzeichnet, daß** sie mit mindestens einer Autoimmunerkrankung aus der Gruppe multiple Sklerose und rheumatoide Polyarthritis assoziiert sind.

13. Nukleotidfragment, das eine Nukleotidsequenz aus der Gruppe (i) der Sequenzen SEQ ID NO: 114, SEQ ID NO: 117, SEQ ID NO: 120; (ii) der zu den Sequenzen (i) komplementären Sequenzen; sowie (iii) der zu den Sequenzen (i) oder (ii) äquivalenten Sequenzen, die über eine beliebige Reihe von 100 aufeinanderfolgenden Monomeren mindestens 50% Homologie mit den Sequenzen (i) bzw. (ii) aufweisen, umfaßt, wobei sich das Nukleotidfragment von der Sequenz HSAC64 des menschlichen Klons RG083M05, der unter der Nummer AC000064 bei der EMBL-Datenbank verfügbar ist, unterscheidet.

14. Nukleotidfragment nach Anspruch 13, **dadurch gekennzeichnet, daß** es eine Nukleotidsequenz aus der Gruppe (iii) der zu den Sequenzen (i) oder (ii) äquivalenten Sequenzen, die über eine beliebige Reihe von 100 aufeinanderfolgenden Monomeren mindestens 70% Homologie mit den Sequenzen (i) bzw. (ii) aufweisen, umfaßt.

15. Nukleotidfragment nach Anspruch 13, das aus einer Nukleotidsequenz aus der Gruppe (i) der Sequenzen SEQ ID NO: 114, SEQ ID NO: 117, SEQ ID NO: 120; (ii) der zu den Sequenzen (i) komplementären Sequenzen; sowie (iii) der zu den Sequenzen (i) oder (ii) äquivalenten Sequenzen, die über eine beliebige Reihe von 100 aufeinanderfolgenden Monomeren mindestens 50% Homologie mit den Sequenzen (i) bzw. (ii) aufweisen, besteht.

16. Nukleotidfragment nach Anspruch 15, **dadurch gekennzeichnet, daß** es aus einer Nukleotidsequenz aus der Gruppe (iii) der zu den Sequenzen (i) oder (ii) äquivalenten Sequenzen, die über eine beliebige Reihe von 100 aufeinanderfolgenden Monomeren mindestens 70% Homologie mit den Sequenzen (i) bzw. (ii) aufweisen, besteht.

17. Nukleotidfragment, das eine Nukleotidsequenz umfaßt, die für ein Polypeptid codiert, das über eine beliebige aufeinanderfolgende Reihe von mindestens 30 Aminosäuren mindestens 50% Homologie mit einer Peptidsequenz aus der Gruppe SEQ ID NO: 115, SEQ ID NO: 118, SEQ ID NO: 121 aufweist, wobei sich das Nukleotidfragment von der Sequenz HSAC64 des menschlichen Klons RG083M05, der unter der Nummer AC000064 bei der EMBL-Datenbank verfügbar ist, unterscheidet.

18. Nukleotidfragment nach Anspruch 17, **dadurch gekennzeichnet, daß** es für ein Polypeptid codiert, das über eine beliebige aufeinanderfolgende Reihe von mindestens 30 Aminosäuren mindestens 70% Homologie mit einer Peptidsequenz aus der Gruppe SEQ ID NO: 115, SEQ ID NO: 118, SEQ ID NO: 121 aufweist.

19. Nukleotidfragment nach Anspruch 17, das aus einer Nukleotidsequenz besteht, die für ein Polypeptid codiert, das über eine beliebige aufeinanderfolgende Reihe von mindestens 30 Aminosäuren mindestens 50% Homologie mit einer Peptidsequenz aus der Gruppe SEQ ID NO: 115, SEQ ID NO: 118 und SEQ ID NO: 121 aufweist.

20. Nukleotidfragment nach Anspruch 19, **dadurch gekennzeichnet, daß** es aus einer Nukleotidsequenz besteht, die für ein Polypeptid codiert, das über eine beliebige aufeinanderfolgende Reihe von mindestens 30 Aminosäuren mindestens 70% Homologie mit einer Peptidsequenz aus der Gruppe SEQ ID NO: 115, SEQ ID NO: 118, SEQ ID NO: 121 aufweist.

21. Gensonde für den Nachweis eines mit multipler Sklerose und/oder rheumatoider Polyarthritis assoziierten Retrovirus, **dadurch gekennzeichnet, daß** sie spezifisch mit einem beliebigen Fragment nach einem der Ansprüche 13 bis 20, das zu dem Genom dieses Retrovirus gehört, hybridisieren kann und daß sie 10 bis 100 Nukleotide aufweist.

22. Sonde nach Anspruch 21, **dadurch gekennzeichnet, daß** sie 10 bis 30 Nukleotide aufweist.

23. Primer für die Amplifikation einer RNA oder einer DNA eines mit multipler Sklerose und/oder rheumatoider Polyarthritis assoziierten Retrovirus mittels Polymerisation, **dadurch gekennzeichnet, daß** er eine Nukleotidsequenz umfaßt, die zu mindestens einem Teil der Nukleotidsequenz eines Fragments nach einem der Ansprüche 13 bis 20, wobei dieses Nukleotidfragment zu dem env-Gen dieses Retrovirus gehört, identisch ist, und dadurch, daß dieser Teil dieser Nukleotidsequenz mindestens 6 Monomere umfaßt.

24. Primer nach Anspruch 23, **dadurch gekennzeichnet, daß** er 10 bis 30 Monomere umfaßt.

25. Primer für die Amplifikation einer RNA oder einer DNA eines mit multipler Sklerose und/oder rheumatoider Polyarthritis assoziierten Retrovirus mittels Polymerisation, **dadurch gekennzeichnet, daß** er eine Nukleotidsequenz umfaßt, die über eine beliebige Reihe von 10 aufeinanderfolgenden Monomeren mindestens 50% Homologie mit einem Fragment nach einem der Ansprüche 13 bis 20, wobei dieses Nukleotidfragment zu dem env-Gen dieses Retrovirus gehört, aufweist, und dadurch, daß diese Nukleotidsequenz 10 bis 30 Monomere umfaßt.

26. Primer nach Anspruch 25, **dadurch gekennzeichnet, daß** er eine Nukleotidsequenz, die über eine beliebige Reihe von 10 aufeinanderfolgenden Monomeren mindestens 70% Homologie mit diesem Fragment aufweist, umfaßt.

27. Primer nach Anspruch 24, **dadurch gekennzeichnet, daß** seine Nukleotidsequenz aus der Gruppe SEQ ID NO: 116 und SEQ ID NO: 119 stammt.

28. Replikations- und/oder Expressionsvektor, der ein Nukleotidfragment nach einem der Ansprüche 13 bis 20 umfaßt.

29. Peptid, das von einem beliebigen offenen Leseraster, das zu einem Nukleotidfragment nach einem der Ansprüche 13 bis 20 gehört, codiert wird.

30. Peptid, das eine Peptidsequenz umfaßt, die mit einer Sequenz aus der Gruppe SEQ ID NO: 115, SEQ ID NO: 118 und SEQ ID NO: 121 stammt, identisch ist.

31. Peptid, das eine Peptidsequenz umfaßt, die über eine beliebige aufeinanderfolgende Reihe von mindestens 30 Aminosäuren mindestens 50% Homologie mit einer Sequenz aus der Gruppe SEQ ID NO: 115, SEQ ID NO: 118 und SEQ ID NO: 121 aufweist.

32. Peptid nach Anspruch 31, **dadurch gekennzeichnet, daß** es eine Peptidsequenz aufweist, die über eine beliebige aufeinanderfolgende Reihe von mindestens 30 Aminosäuren mindestens 70% Homologie mit einer Sequenz aus der Gruppe SEQ ID NO: 115, SEQ ID NO: 118 und SEQ ID NO: 121 aufweist.

33. Diagnostische, prophylaktische oder therapeutische Zusammensetzung, die ein Nukleotidfragment nach einem der Ansprüche 13 bis 20 umfaßt.

34. Verfahren für den Nachweis eines mit multipler Sklerose und/oder rheumatoider Polyarthritis assoziierten Retrovirus in einer biologischen Probe, **dadurch gekennzeichnet, daß** man eine RNA und/oder eine DNA, von der angenommen wird, daß sie zu diesem Retrovirus gehört oder davon stammt, oder deren komplementäre RNA bzw. DNA mit einer Zusammensetzung, die ein Nukleotidfragment nach einem der Ansprüche 13 bis 20 umfaßt, in Kontakt bringt.

## Claims

1. Nucleic material, in isolated or purified state, comprising a nucleotide sequence chosen from the group which consists of (i) the sequences SEQ ID NO: 114, SEQ ID NO: 117 and SEQ ID NO: 120; (ii) the sequences complementary to sequences (i); and (iii) the sequences equivalent to sequences (i) or (ii) having, for every series of 100 contiguous monomers, at least 50% homology with sequences (i) or (ii) respectively, said nucleic material being different from the sequence HSAC64 of the human clone RG083M05 accessible on the EMBL database under the number AC000064.

2. Nucleic material according to Claim 1, **characterized in that** it comprises a nucleotide sequence chosen from the group (iii) of sequences equivalent to sequences (i) or (ii) having, for every series of 100 contiguous monomers, at least 70% homology with sequences (i) or (ii) respectively.

3. Nucleic material, in isolated or purified state, encoding a polypeptide having, for every contiguous series of at least 30 amino acids, at least 50% homology with a peptide sequence chosen from the group which consists of SEQ ID NO: 115, SEQ ID NO: 118 and SEQ ID NO: 121, said nucleic material being different from the sequence HSAC64 of the human clone RG083M05 accessible on the EMBL database under the number AC000064.

4. Nucleic material according to Claim 3, **characterized in that** it encodes a polypeptide having, for every contiguous series of at least 30 amino acids, at least 70% homology with a peptide sequence chosen from the group which consists of SEQ ID NO: 115, SEQ ID NO: 118 and SEQ ID NO: 121.

5. Retroviral nucleic material, in which the env gene comprises a nucleotide sequence chosen from the group which consists of SEQ ID NO: 114, SEQ ID NO: 117 and SEQ ID NO: 120, and their complementary sequences.

6. Retroviral nucleic material, in which the env gene comprises a nucleotide sequence which starts at nucleotide 1 of SEQ ID NO: 117 and ends at nucleotide 233 of SEQ ID NO: 114.

7. Retroviral nucleic material, in which the env gene encodes a polypeptide having, for every contiguous series of at least 30 amino acids, at least 50% homology with the sequence SEQ ID NO: 118.

8. Nucleic material according to Claim 7, **characterized in that** its env gene encodes a polypeptide having, for every contiguous series of at least 30 amino acids, at least 70% homology with the sequence SEQ ID NO: 118.

9. Retroviral nucleic material in which the U3R region of the 3' LTR comprises a nucleotide sequence which ends at nucleotide 617 of SEQ ID NO: 114.

10. Retroviral nucleic material in which the RU5 region of the 5' LTR comprises a nucleotide sequence which starts at nucleotide 755 of SEQ ID NO: 120.

11. Retroviral nucleic material comprising a sequence which starts at nucleotide 755 of SEQ ID NO: 120 and which ends at nucleotide 617 of SEQ ID NO: 114.

12. Retroviral nucleic material according to any one of the preceding claims, **characterized in that** it is associated with at least one autoimmune disease such as multiple sclerosis or rheumatoid arthritis.

13. Nucleotide fragment comprising a nucleotide sequence chosen from the group which consists of (i) the sequences SEQ ID NO: 114, SEQ ID NO: 117 and SEQ ID NO: 120; (ii) the sequences complementary to sequences (i); and (iii) the sequences equivalent to sequences (i) or (ii) having, for every series of 100 contiguous monomers, at least 50% homology with sequences (i) or (ii) respectively, said nucleotide fragment being different from the sequence HSAC64 of the human clone RG083M05 accessible on the EMBL database under the number AC000064.

14. Nucleotide fragment according to Claim 13, **characterized in that** it comprises a nucleotide sequence chosen from the group (iii) of sequences equivalent to sequences (i) or (ii) having, for every series of 100 contiguous monomers, at least 70% homology with sequences (i) or (ii) respectively.

15. Nucleotide fragment according to Claim 13, consisting of a nucleotide sequence chosen from the group which consists of (i) the sequences SEQ ID NO: 114, SEQ ID NO: 117 and SEQ ID NO: 120; (ii) the sequences complementary to sequences (i); and (iii) the sequences equivalent to sequences (i) or (ii) having, for every series of 100 contiguous monomers, at least 50% homology with sequences (i) or (ii) respectively.

16. Nucleotide fragment according to Claim 15, **characterized in that** it consists of a nucleotide sequence chosen from the group (iii) of sequences equivalent to sequences (i) or (ii) having, for every series of 100 contiguous monomers, at least 70% homology with sequences (i) or (ii) respectively.

17. Nucleotide fragment comprising a nucleotide sequence encoding a polypeptide having, for every contiguous series of at least 30 amino acids, at least 50% homology with a peptide sequence chosen from the group which consists of SEQ ID NO: 115, SEQ ID NO: 118 and SEQ ID NO: 121, said nucleotide fragment being different from the sequence HSAC64 of the human clone RG083M05 accessible on the EMBL database under the number AC000064.

18. Nucleotide fragment according to Claim 17, **characterized in that** it encodes a polypeptide having, for every contiguous series of at least 30 amino acids, at least 70% homology with a peptide sequence chosen from the group which consists of SEQ ID NO: 115, SEQ ID NO: 118 and SEQ ID NO: 121.

19. Nucleotide fragment according to Claim 17, consisting of a nucleotide sequence encoding a polypeptide having, for every contiguous series of at least 30 amino acids, at least 50% homology with a peptide sequence chosen from the group which consists of SEQ ID NO: 115, SEQ ID NO: 118 and SEQ ID NO: 121.

20. Nucleotide fragment according to Claim 19, **characterized in that** it consists of a nucleotide sequence encoding a polypeptide having, for every contiguous series of at least 30 amino acids, at least 70% homology with a peptide sequence chosen from the group which consists of SEQ ID NO: 115, SEQ ID NO: 118 and SEQ ID NO: 121.

21. Nucleic probe for the detection of a retrovirus associated with multiple sclerosis and/or rheumatoid arthritis, **characterized in that** it is capable of hybridizing specifically with any fragment according to any one of Claims 13 to 20, belonging to the genome of said retrovirus, and **in that** it possesses from 10 to 100 nucleotides.

22. Probe according to Claim 21, **characterized in that** it possesses from 10 to 30 nucleotides.

23. Primer for the amplification, by polymerization, of an RNA or of a DNA of a retrovirus associated with multiple sclerosis and/or rheumatoid arthritis, **characterized in that** it comprises a nucleotide sequence identical to at least a portion of the nucleotide sequence of a fragment according to any one of Claims 13 to 20, said nucleotide fragment belonging to the env gene of said retrovirus, and **in that** said portion of the nucleotide sequence comprises at least 6 monomers.

24. Primer according to Claim 23, **characterized in that** it comprises from 10 to 30 monomers.

25. Primer for the amplification, by polymerization, of an RNA or of a DNA of a retrovirus associated with multiple sclerosis and/or rheumatoid arthritis, **characterized in that** it comprises a nucleotide sequence having, for every series of 10 contiguous monomers, at least 50% homology with a fragment according to any one of Claims 13 to 20, said nucleotide fragment belonging to the env gene of said retrovirus, and **in that** said nucleotide sequence comprises from 10 to 30 monomers.

26. Primer according to Claim 25, **characterized in that** it comprises a nucleotide sequence having, for every series of 10 contiguous monomers, at least 70% homology with said fragment.

27. Primer according to Claim 24, **characterized in that** its nucleotide sequence is chosen from SEQ ID NO: 116 and SEQ ID NO: 119.

28. Replication and/or expression vector, comprising a nucleotide fragment according to any one of Claims 13 to 20.

29. Peptide encoded by any open reading frame belonging to a nucleotide fragment according to any one of Claims 13 to 20.

30. Peptide comprising a peptide sequence identical to a sequence chosen from SEQ ID NO: 115, SEQ ID NO: 118 and SEQ ID NO: 121.

31. Peptide comprising a peptide sequence which, for every contiguous series of at least 30 amino acids, has at least 50% homology with a sequence chosen from SEQ ID NO: 115, SEQ ID NO: 118 and SEQ ID NO: 121.

32. Peptide according to Claim 31, **characterized in that** it comprises a peptide sequence which, for every contiguous series of at least 30 amino acids, has at least 70% homology with a sequence chosen from SEQ ID NO: 115, SEQ ID NO: 118 and SEQ ID NO: 121.

33. Diagnostic, prophylactic or therapeutic composition comprising a nucleotide fragment according to any one of Claims 13 to 20.

34. Method for detecting a retrovirus associated with multiple sclerosis and/or rheumatoid arthritis, in a biological sample, **characterized in that** an RNA and/or a DNA assumed to belong to or obtained from said retrovirus, or their complementary RNA and/or DNA, is brought into contact with a composition comprising a nucleotide fragment according to any one of Claims 13 to 20.


### LISTE DE SEQUENCES

(2) INFORMATIONS POUR LA SEQ ID NO: 68:
   (i) CARACTERISTIQUES DE LA SEQUENCE:
      (A) LONGUEUR: 34 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 68:
   GACTCGCTGC AGATCGATTT TTTTTTTTTT TTTT 34
(2) INFORMATIONS POUR LA SEQ ID NO: 69:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 69:
   GCCATCAAGC CACCCAAGAA CTCTTAACTT 30
(2) INFORMATIONS POUR LA SEQ ID NO: 70:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 70:
   CCAATAGCCA GACCATTATA TACACTAATT 30
(2) INFORMATIONS POUR LA SEQ ID NO: 112:
   (i) CARACTERISTIQUES DE LA SEQUENCE:
      (A) LONGUEUR: 310 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 112:
   GCTTATAGAA GGACCCCTAG TATGGGGTAA TCCCCTCTGG GAAACCAAGC CCCAGTACTC    60
   AGCAGGAAAA ATAGAATAGG AAACCTCACA AGGACATACT TTCCTCCCCT CCAGATGGCT   120
   AGCCACTGAG GAAGGAAAAA TACTTTCACC TGCAGCTAAC CAACAGAAAT TACTTAAAAC   180
   CCTTCACCAA ACCTTCCACT TAGGCATTGA TAGCACCCAT CAGATGGCCA AATTATTATT   240
   TACTGGACCA GGCCTTTTCA AAACTATCAA GAAGATAGTC AGGGGCTGTG AAGTGTGCCA   300
   AAGAAATAAT                                                          310
(2) INFORMATIONS POUR LA SEQ ID NO: 113:
   (i) CARACTERISTIQUES DE LA SEQUENCE:
      (A) LONGUEUR: 103 acides aminéés
      (B) TYPE: acide aminéé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 113:
   Leu Ile Glu Gly Pro Leu Val Trp Gly Asn Pro Leu Trp Glu Thr Lys
   1               5                   10                  15
   Pro Gln Tyr Ser Ala Gly Lys Ile Glu Xaa Glu Thr Ser Gln Gly His
               20                  25                  30
   Thr Phe Leu Pro Ser Arg Trp Leu Ala Thr Glu Glu Gly Lys Ile Leu
           35                  40                  45
   Ser Pro Ala Ala Asn Gln Gln Lys Leu Leu Lys Thr Leu His Gln Thr
       50                  55                  60
   Phe His Leu Gly Ile Asp Ser Thr His Gln Met Ala Lys Leu Leu Phe
   65                  70                  75                  80
   Thr Gly Pro Gly Leu Phe Lys Thr Ile Lys Lys Ile Val Arg Gly Cys
                   85                  90                  95
   Glu Val Cys Gln Arg Asn Asn
               100
(2) INFORMATIONS POUR LA SEQ ID NO: 114:
   (i) CARACTERISTIQUES DE LA SEQUENCE:
      (A) LONGUEUR: 635 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 114:
   CCCTGTATCT TTAACCTCCT TGTTAAGTTT GTCTCTTCCA GAATCAAAAC TGTAAAACTA   60
   CAAATTGTTC TTCAAATGGA GCACCAGATG GAGTCCATGA CTAAGATCCA CCGTGGACCC  120
   CTGGACCGGC CTGCTAGCCC ATGCTCCGAT GTTAATGACA TTGAAGGCAC CCCTCCCGAG  180
   GAAATCTCAA CTGCACAACC CCTACTATGC CCCAATTCAG CGGGAAGCAG TTAGAGCGGT  240
   CATCAGCCAA CCTCCCCAAC AGCACTTGGG TTTTCCTGTT GAGAGGGGGG ACTGAGAGAC  300
   AGGACTAGCT GGATTTCCTA GGCCAACGAA GAATCCCTAA GCCTAGCTGG GAAGGTGACT  360
   GCATCCACCT CTAAACATGG GGCTTGCAAC TTAGCTCACA CCCGACCAAT CAGAGAGCTC  420
   ACTAAAATGC TAATTAGGCA AAAATAGGAG GTAAAGAAAT AGCCAATCAT CTATTGCCTG  480
   AGAGCACAGC GGGAGGGACA AGGATCGGGA TATAAACCCA GGCATTCGAG CCGGCAACGG  540
   CAACCCCCTT TGGGTCCCCT CCCTTTGTAT GGGCGCTCTG TTTTCACTCT ATTTCACTCT  600
   ATTAAATCTT GCAACTGAAA AAAAAAAAAA AAAAA                             635
(2) INFORMATIONS POUR LA SEQ ID NO: 115:
   (i) CARACTERISTIQUES DE LA SEQUENCE:
      (A) LONGUEUR: 77 acides aminéés
      (B) TYPE: acide aminéé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 115:
   Pro Cys Ile Phe Asn Leu Leu Val Lys Phe Val Ser Ser Arg Ile Lys
   1               5                   10                  15
   Thr Val Lys Leu Gln Ile Val Leu Gln Met Glu His Gln Met Glu Ser
               20                  25                  30
   Met Thr Lys Ile His Arg Gly Pro Leu Asp Arg Pro Ala Ser Pro Cys
           35                  40                  45
   Ser Asp Val Asn Asp Ile Glu Gly Thr Pro Pro Glu Glu Ile Ser Thr
       50                  55                  60
   Ala Gln Pro Leu Leu Cys Pro Asn Ser Ala Gly Ser Ser
   65                  70                  75
(2) INFORMATIONS POUR LA SEQ ID NO: 116:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 116:
   TGGGGTTCCA TTTGTAAGAC CATCTGTAGC TT 32
(2) INFORMATIONS POUR LA SEQ ID NO: 117:
   (i) CARACTERISTIQUES DE LA SEQUENCE:
      (A) LONGUEUR: 1481 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 117:
   ATGGCCCTCC CTTATCATAC TTTTCTCTTT ACTGTTCTCT TACCCCCTTT CGCTCTCACT 60
   GCACCCCCTC CATGCTGCTG TACAACCAGT AGCTCCCCTT ACCAAGAGTT TCTATGAAGA 120
   ACGCGGCTTC CTGGAAATAT TGATGCCCCA TCATATAGGA GTTTATCTAA GGGAAACTCC 180
   ACCTTCACTG CCCACACCCA TATGCCCCGC AACTGCTATA ACTCTGCCAC TCTTTGCATG 240
   CATGCAAATA CTCATTATTG GACAGGGAAA ATGATTAATC CTAGTTGTCC TGGAGGACTT 300
   GGAGCCACTG TCTGTTGGAC TTACTTCACC CATACCAGTA TGTCTGATGG GGGTGGAATT 360
   CAAGGTCAGG CAAGAGAAAA ACAAGTAAAG GAAGCAATCT CCCAACTGAC CCGGGGACAT 420
   AGCACCCCTA GCCCCTACAA AGGACTAGTT CTCTCAAAAC TACATGAAAC CCTCCGTACC 480
   CATACTCGCC TGGTGAGCCT ATTTAATACC ACCCTCACTC GGCTCCATGA GGTCTCAGCC 540
   CAAAACCCTA CTAACTGTTG GATGTGCCTC CCCCTGCACT TCAGGCCATA CATTTCAATC 600
   CCTGTTCCTG AACAATGGAA CAACTTCAGC ACAGAAATAA ACACCACTTC CGTTTTAGTA 660
   GGACCTCTTG TTTCCAATCT GGAAATAACC CATACCTCAA ACCTCACCTG TGTAAAATTT 720
   AGCAATACTA TAGACACAAC CAGCTCCCAA TGCATCAGGT GGGTAACACC TCCCACACGA 780
   ATAGTCTGCC TACCCTCAGG AATATTTTTT GTCTGTGGTA CCTCAGCCTA TCATTGTTTG 840
   AATGGCTCTT CAGAATCTAT GTGCTTCCTC TCATTCTTAG TGCCCCCTAT GACCATCTAC 900
   ACTGAACAAG ATTTATACAA TCATGTCGTA CCTAAGCCCC ACAACAAAAG AGTACCCATT 960
   CTTCCTTTTG TTATCAGAGC AGGAGTGCTA GGCAGACTAG GTACTGGCAT TGGCAGTATC 1020
   ACAACCTCTA CTCAGTTCTA CTACAAACTA TCTCAAGAAA TAAATGGTGA CATGGAACAG 1080
   GTCACTGACT CCCTGGTCAC CTTGCAAGAT CAACTTAACT CCCTAGCAGC AGTAGTCCTT 1140
   CAAAATCGAA GAGCTTTAGA CTTGCTAACC GCCAAAAGAG GGGGAACCTG TTTATTTTTA 1200
   GGAGAAGAAC GCTGTTATTA TGTTAATCAA TCCAGAATTG TCACTGAGAA AGTTAAAGAA 1260
   ATTCGAGATC GAATACAATG TAGAGCAGAG GAGCTTCAAA ACACCGAACG CTGGGGCCTC 1320
   CTCAGCCAAT GGATGCCCTG GGTTCTCCCC TTCTTAGGAC CTCTAGCAGC TCTAATATTG 1380
   TTACTCCTCT TTGGACCCTG TATCTTTAAC CTCCTTGTTA AGTTTGTCTC TTCCAGAATT 1440
   GAAGCTGTAA AGCTACAGAT GGTCTTACAA ATGGAACCCC A 1481
(2) INFORMATIONS POUR LA SEQ ID NO: 118:
   (i) CARACTERISTIQUES DE LA SEQUENCE:
      (A) LONGUEUR: 493 acides aminéés
      (B) TYPE: acide aminéé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 118:
   Met Ala Leu Pro Tyr His Thr Phe Leu Phe Thr Val Leu Leu Pro Pro
   1 5 10 15
   Phe Ala Leu Thr Ala Pro Pro Pro Cys Cys Cys Thr Thr Ser Ser Ser
   20 25 30
   Pro Tyr Gln Glu Phe Leu Xaa Arg Thr Arg Leu Pro Gly Asn Ile Asp
   35 40 45
   Ala Pro Ser Tyr Arg Ser Leu Ser Lys Gly Asn Ser Thr Phe Thr Ala
   50 55 60
   His Thr His Met Pro Arg Asn Cys Tyr Asn Ser Ala Thr Leu Cys Met
   65 70 75 80
   His Ala Asn Thr His Tyr Trp Thr Gly Lys Met Ile Asn Pro Ser Cys
   85 90 95
   Pro Gly Gly Leu Gly Ala Thr Val Cys Trp Thr Tyr Phe Thr His Thr
   100 105 110
   Ser Met Ser Asp Gly Gly Gly Ile Gln Gly Gln Ala Arg Glu Lys Gln
   115 120 125
   Val Lys Glu Ala Ile Ser Gln Leu Thr Arg Gly His Ser Thr Pro Ser
   130 135 140
   Pro Tyr Lys Gly Leu Val Leu Ser Lys Leu His Glu Thr Leu Arg Thr
   145 150 155 160
   His Thr Arg Leu Val Ser Leu Phe Asn Thr Thr Leu Thr Arg Leu His
   165 170 175
   Glu Val Ser Ala Gln Asn Pro Thr Asn Cys Trp Met Cys Leu Pro Leu
   180 185 190
   His Phe Arg Pro Tyr Ile Ser Ile Pro Val Pro Glu Gln Trp Asn Asn
   195 200 205
   Phe Ser Thr Glu Ile Asn Thr Thr Ser Val Leu Val Gly Pro Leu Val
   210 215 220
   Ser Asn Leu Glu Ile Thr His Thr Ser Asn Leu Thr Cys Val Lys Phe
   225 230 235 240
   Ser Asn Thr Ile Asp Thr Thr Ser Ser Gln Cys Ile Arg Trp Val Thr
   245 250 255
   Pro Pro Thr Arg Ile Val Cys Leu Pro Ser Gly Ile Phe Phe Val Cys
   260 265 270
   Gly Thr Ser Ala Tyr His Cys Leu Asn Gly Ser Ser Glu Ser Met Cys
   275 280 285
   Phe Leu Ser Phe Leu Val Pro Pro Met Thr Ile Tyr Thr Glu Gln Asp
   290 295 300
   Leu Tyr Asn His Val Val Pro Lys Pro His Asn Lys Arg Val Pro Ile
   305 310 315 320
   Leu Pro Phe Val Ile Arg Ala Gly Val Leu Gly Arg Leu Gly Thr Gly
   325 330 335
   Ile Gly Ser Ile Thr Thr Ser Thr Gln Phe Tyr Tyr Lys Leu Ser Gln
   340 345 350
   Glu Ile Asn Gly Asp Met Glu Gln Val Thr Asp Ser Leu Val Thr Leu
   355 360 365
   Gln Asp Gln Leu Asn Ser Leu Ala Ala Val Val Leu Gln Asn Arg Arg
   370 375 380
   Ala Leu Asp Leu Leu Thr Ala Lys Arg Gly Gly Thr Cys Leu Phe Leu
   385 390 395 400
   Gly Glu Glu Arg Cys Tyr Tyr Val Asn Gln Ser Arg Ile Val Thr Glu
   405 410 415
   Lys Val Lys Glu Ile Arg Asp Arg Ile Gln Cys Arg Ala Glu Glu Leu
   420 425 430
   Gln Asn Thr Glu Arg Trp Gly Leu Leu Ser Gln Trp Met Pro Trp Val
           435                     440                     445
   Leu Pro Phe Leu Gly Pro Leu Ala Ala Leu Ile Leu Leu Leu Leu Phe
       450                     455                     460
   Gly Pro Cys Ile Phe Asn Leu Leu Val Lys Phe Val Ser Ser Arg Ile
   465                 470                     475                 480
   Glu Ala Val Lys Leu Gln Met Val Leu Gln Met Glu Pro
                   485                     490
(2) INFORMATIONS POUR LA SEQ ID NO: 119:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 119:
   TCAAAATCGA AGAGCTTTAG ACTTGCTAAC CG                                  32
(2) INFORMATIONS POUR LA SEQ ID NO: 120:
   (i) CARACTERISTIQUES DE LA SEQUENCE:
      (A) LONGUEUR: 1329 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 120:
   TCAAAATCGA AGAGCTTTAG ACTTGCTAAC CGCCAAAAGA GGGGGAACCT GTTTATTTTT    60
   AGGGGAAGAA TGCTGTTAGT ATGTTAATCA ATCTGGAATC ATTACTGAGA AAGTTAAAGA   120
   AATTTGAGAT CGAATATAAT GTAGAGCAGA GGACCTTCAA AACACTGCAC CCTGGGGCCT   180
   CCTCAGCCAA TGGATGCCCT GGACTCTCCC CTTCTTAGGA CCTCTAGCAG CTATAATATT   240
   TTTACTCCTC TTTGGACCCT GTATCTTCAA CTTCCTTGTT AAGTTTGTCT CTTCCAGAAT   300
   TGAAGCTGTA AAGCTACAAA TAGTTCTTCA AATGGAACCC CAGATGCAGT CCATGACTAA   360
   AATCTACCGT GGACCCCTGG ACCGGCCTGC TAGACTATGC TCTGATGTTA ATGACATTGA   420
   AGTCACCCCT CCCGAGGAAA TCTCAACTGC ACAACCCCTA CTACACTCCA ATTCAGTAGG   480
   AAGCAGTTAG AGCAGTTGTC AGCCAACCTC CCCAACAGTA CTTGGGTTTT CCTGTTGAGA   540
   GGGTGGACTG AGAGACAGGA CTAGCTGGAT TTCCTAGGCT GACTAAGAAT CCCNAAGCCT   600
   ANCTGGGAAG GTGACCGCAT CCATCTTTAA ACATGGGGCT TGCAACTTAG CTCACACCCG   660
   ACCAATCAGA GAGCTCACTA AAATGCTAAT CAGGCAAAAA CAGGAGGTAA AGCAATAGCC   720
   AATCATCTAT TGCCTGAGAG CACAGCGGGA AGGACAAGGA TTGGGATATA AACTCAGGCA   780
   TTCAAGCCAG CAACAGCAAC CCCCTTTGGG TCCCCTCCCA TTGTATGGGA GCTCTGTTTT   840
   CACTCTATTT CACTCTATTA AATCATGCAA CTGCACTCTT CTGGTCCGTG TTTTTTATGG   900
   CTCAAGCTGA GCTTTTGTTC GCCATCCACC ACTGCTGTTT GCCACCGTCA CAGACCCGCT   960
   GCTGACTTCC ATCCCTTTGG ATCCAGCAGA GTGTCCACTG TGCTCCTGAT CCAGCGAGGT  1020
   ACCCATTGCC ACTCCCGATC AGGCTAAAGG CTTGCCATTG TTCCTGCATG GCTAAGTGCC  1080
   TGGGTTTGTC CTAATAGAAC TGAACACTGG TCACTGGGTT CCATGGTTCT CTTCCATGAC  1140
   CCACGGCTTC TAATAGAGCT ATAACACTCA CCGCATGGCC CAAGATTCCA TTCCTTGGTA  1200
   TCTGTGAGGC CAAGAACCCC AGGTCAGAGA ANGTGAGGCT TGCCACCATT TGGGAAGTGG  1260
   CCCACTGCCA TTTTGGTAGC GGCCCACCAC CATCTTGGGA GCTGTGGGAG CAAGGATCCC  1320
   CCAGTAACA                                                          1329
(2) INFORMATIONS POUR LA SEQ ID NO: 121:
   (i) CARACTERISTIQUES DE LA SEQUENCE:
      (A) LONGUEUR: 162 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 121:
   Gln Asn Arg Arg Ala Leu Asp Leu Leu Thr Ala Lys Arg Gly Gly Thr
   1               5                   10                  15
   Cys Leu Phe Leu Gly Glu Glu Cys Cys Xaa Tyr Val Asn Gln Ser Gly
               20                  25                  30
   Ile Ile Thr Glu Lys Val Lys Glu Ile Xaa Asp Arg Ile Xaa Cys Arg
           35                  40                  45
   Ala Glu Asp Leu Gln Asn Thr Ala Pro Trp Gly Leu Leu Ser Gln Trp
       50                  55                  60
   Met Pro Trp Thr Leu Pro Phe Leu Gly Pro Leu Ala Ala Ile Ile Phe
   65                  70                  75                  80
   Leu Leu Leu Phe Gly Pro Cys Ile Phe Asn Phe Leu Val Lys Phe Val
                   85                  90                  95
   Ser Ser Arg Ile Glu Ala Val Lys Leu Gln Ile Val Leu Gln Met Glu
               100                 105                 110
   Pro Gln Met Gln Ser Met Thr Lys Ile Tyr Arg Gly Pro Leu Asp Arg
   115 120 125
   Pro Ala Arg Leu Cys Ser Asp Val Asn Asp Ile Glu Val Thr Pro Pro
   130 135 140
   Glu Glu Ile Ser Thr Ala Gln Pro Leu Leu His Ser Asn Ser Val Gly
   145 150 155 160
   Ser Ser
(2) INFORMATIONS POUR LA SEQ ID NO: 122:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 122:
   GGCATTGATA GCACCCATCA G 21
(2) INFORMATIONS POUR LA SEQ ID NO: 123:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 123:
   CATGTCACCA GGGTGGAATA G 21
(2) INFORMATIONS POUR LA SEQ ID NO: 124:
   (i) CARACTERISTIQUES DE LA SEQUENCE:
      (A) LONGUEUR: paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 124:
(2) INFORMATIONS POUR LA SEQ ID NO: 124:
   (i) CARACTERISTIQUES DE LA SEQUENCE:
      (A) LONGUEUR: 758 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 124:
   GGCATTGATA GCACCCATCA GATGGCCAAA TCATTATTTA CTGGACCAGG CCTTTTCAAA 60
   ACTATCAAGC AGATAGGGCC CGTGAAGCAT GCCAAAGAAA TAATCCCCTG CCTTATCGCC 120
   ATGTTCCTTC AGGAGAACAA AGAACAGGCC ATTACCCAGG GGAAGACTGG CAACTAGATT 180
   TTACCCACAT GGCCAAATGT CAGGGATTTC AGCATCTACT AGTCTGGGCA GATACTTTCA 240
   CTGGTTGGGT GGAGTCTTCT CCTTGTAGGA CAGAAAAGAC CCAAGAGGTA ATAAAGGCAC 300
   TAATGAAATA ATTCCCAGAT TTGGACTTCC CCCAGGATTA CAGGGTGACA ATGGCCCCGC 360
   TTTCAAGGCT GCAGTAACCC AGGGAGTATC CCAGGTGTTA GGCATACAAT ATCACTTACA 420
   CTGTGCCTGG AGGCCACAAT CCTCCAGAAA AGTCAAGAAA ATGAATGAAA CACTCAAAGA 480
   TCTAAAAAAG CTAACCCAAG AAACCCACAT TGCATGACCT GTTCTGTTGC CTATAACCTT 540
   ACTAAGAATC CATAACTATC CCCCAAAAAG CAGGACTTAG CCCATACGAG ATGCTATATG 600
   GATGGCCTTT CCTAACCAAT GACCTTGTGC TTGACTGAGA AATGGCCAAC TTAGTTGCAG 660
   ACATCACCTC CTTAGCCAAA TATCAACAAG TTCTTAAAAC ATCACAGGGA ACCTGTCCCC 720
   GAGAGGAGGG AAAGGAACTA TTCCACCCTG GTGACATG 758
(2) INFORMATIONS POUR LA SEQ ID NO: 126:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 126:
   CGGACATCCA AAGTGATGGG AAACG 25
(2) INFORMATIONS POUR LA SEQ ID NO: 127:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 127:
   GGACAGGAAA GTAAGACTGA GAAGGC 26
(2) INFORMATIONS POUR LA SEQ ID NO: 128:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 128:
   CCTAGAACGT ATTCTGGAGA ATTGGG 26
(2) INFORMATIONS POUR LA SEQ ID NO: 129:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 129:
   TGGCTCTCAA TGGTCAAACA TACCCG 26
(2) INFORMATIONS POUR LA SEQ ID NO: 130:
   (i) CARACTERISTIQUES DE LA SEQUENCE:
      (A) LONGUEUR: 1511 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 130:
   CCTAGAACGT ATTCTGGAGA ATTGGGACCA ATGTGACACT CAGACGCTAA GAAAGAAACG 60
   ATTTATATTC TTCTGCAGTA CCGCCTGGCC ACAATATCCT CTTCAAGGGA GAGAAACCTG 120
   GCTTCCTGAG GGAAGTATAA ATTATAACAT CATCTTACAG CTAGACCTCT TCTGTAGAAA 180
   GGAGGGCAAA TGGAGTGAAG TGCCATATGT GCAAACTTTC TTTTCATTAA GAGACAACTC 240
   ACAATTATGT AAAAAGTGTG GTTTATGCCC TACAGGAAGC CCTCAGAGTC CACCTCCCTA 300
   CCCCAGCGTC CCCTCCCCGA CTCCTTCCTC AACTAATAAG GACCCCCCTT TAACCCAAAC 360
   GGTCCAAAAG GAGATAGACA AAGGGGTAAA CAATGAACCA AAGAGTGCCA ATATTCCCCG 420
   ATTATGCCCC CTCCAAGCAG TGAGAGGAGG AGAATTCGGC CCAGCCAGAG TGCCTGTACC 480
   TTTTTCTCTC TCAGACTTAA AGCAAATTAA AATAGACCTA GGTAAATTCT CAGATAACCC 540
   TGACGGCTAT ATTGATGTTT TACAAGGGTT AGGACAATCC TTTGATCTGA CATGGAGAGA 600
   TATAATGTTA CTACTAAATC AGACACTAAC CCCAAATGAG AGAAGTGCCG CTGTAACTGC 660
   AGCCCGAGAG TTTGGCGATC TTTGGTATCT CAGTCAGGCC AACAATAGGA TGACAACAGA 720
   GGAAAGAACA ACTCCCACAG GCCAGCAGGC AGTTCCCAGT GTAGACCCTC ATTGGGACAC 780
   AGAATCAGAA CATGGAGATT GGTGCCACAA ACATTTGCTA ACTTGCGTGC TAGAAGGACT 840
   GAGGAAAACT AGGAAGAAGC CTATGAATTA CTCAATGATG TCCACTATAA CACAGGGAAA 900
   GGAAGAAAAT CTTACTGCTT TTCTGGACAG ACTAAGGGAG GCATTGAGGA AGCATACCTC 960
   CCTGTCACCT GACTCTATTG AAGGCCAACT AATCTTAAAG GATAAGTTTA TCACTCAGTC 1020
   AGCTGCAGAC ATTAGAAAAA ACTTCAAAAG TCTGCCTTAG GCCCGGAGCA GAACTTAGAA 1080
   ACCCTATTTA ACTTGGCATC CTCAGTTTTT TATAATAGAG ATCAGGAGGA GCAGGCGAAA 1140
   CGGGACAAAC GGGATAAAAA AAAAAGGGGG GGTCCACTAC TTTAGTCATG GCCCTCAGGC 1200
   AAGCAGACTT TGGAGGCTCT GCAAAAGGGA AAAGCTGGGC AAATCAAATG CCTAATAGGG 1260
   CTGGCTTCCA GTGCGGTCTA CAAGGACACT TTAAAAAAGA TTATCCAAGT AGAAATAAGC 1320
   CGCCCCCTTG TCCATGCCCC TTACGTCAAG GGAATCACTG GAAGGCCCAC TGCCCCAGGG 1380
   GATGAAGATA CTCTGAGTCA GAAGCCATTA ACCAGATGAT CCAGCAGCAG GACTGAGGGT 1440
   GCCCGGGGCG AGCGCCAGCC CATGCCATCA CCCTCACAGA GCCCCGGGTA TGTTTGACCA 1500
   TTGAGAGCCA A 1511
(2) INFORMATIONS POUR LA SEQ ID NO: 131:
   (i) CARACTERISTIQUES DE LA SEQUENCE:
      (A) LONGUEUR: 352 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 131:
   Leu Glu Arg Ile Leu Glu Asn Trp Asp Gln Cys Asp Thr Gln Thr Leu
   1 5 10 15
   Arg Lys Lys Arg Phe Ile Phe Phe Cys Ser Thr Ala Trp Pro Gln Tyr
   20 25 30
   Pro Leu Gln Gly Arg Glu Thr Trp Leu Pro Glu Gly Ser Ile Asn Tyr
   35 40 45
   Asn Ile Ile Leu Gln Leu Asp Leu Phe Cys Arg Lys Glu Gly Lys Trp
   50 55 60
   Ser Glu Val Pro Tyr Val Gln Thr Phe Phe Ser Leu Arg Asp Asn Ser
   65 70 75 80
   Gln Leu Cys Lys Lys Cys Gly Leu Cys Pro Thr Gly Ser Pro Gln Ser
   85 90 95
   Pro Pro Pro Tyr Pro Ser Val Pro Ser Pro Thr Pro Ser Ser Thr Asn
   100 105 110
   Lys Asp Pro Pro Leu Thr Gln Thr Val Gln Lys Glu Ile Asp Lys Gly
   115 120 125
   Val Asn Asn Glu Pro Lys Ser Ala Asn Ile Pro Arg Leu Cys Pro Leu
   130 135 140
   Gln Ala Val Arg Gly Gly Glu Phe Gly Pro Ala Arg Val Pro Val Pro
   145 150 155 160
   Phe Ser Leu Ser Asp Leu Lys Gln Ile Lys Ile Asp Leu Gly Lys Phe
   165 170 175
   Ser Asp Asn Pro Asp Gly Tyr Ile Asp Val Leu Gln Gly Leu Gly Gln
   180 185 190
   Ser Phe Asp Leu Thr Trp Arg Asp Ile Met Leu Leu Leu Asn Gln Thr
   195 200 205
   Leu Thr Pro Asn Glu Arg Ser Ala Ala Val Thr Ala Ala Arg Glu Phe
   210 215 220
   Gly Asp Leu Trp Tyr Leu Ser Gln Ala Asn Asn Arg Met Thr Thr Glu
   225 230 235 240
   Glu Arg Thr Thr Pro Thr Gly Gln Gln Ala Val Pro Ser Val Asp Pro
   245 250 255
   His Trp Asp Thr Glu Ser Glu His Gly Asp Trp Cys His Lys His Leu
   260 265 270
   Leu Thr Cys Val Leu Glu Gly Leu Arg Lys Thr Arg Lys Lys Pro Met
   275 280 285
   Asn Tyr Ser Met Met Ser Thr Ile Thr Gln Gly Lys Glu Glu Asn Leu
   290 295 300
   Thr Ala Phe Leu Asp Arg Leu Arg Glu Ala Leu Arg Lys His Thr Ser
   305 310 315 320
   Leu Ser Pro Asp Ser Ile Glu Gly Gln Leu Ile Leu Lys Asp Lys Phe
   325 330 335
   Ile Thr Gln Ser Ala Ala Asp Ile Arg Lys Asn Phe Lys Ser Leu Pro
   340 345 350
(2) INFORMATIONS POUR LA SEQ ID NO: 132:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 132:
   TGCTGGAATT CGGGATCCTA GAACGTATTC 30
(2) INFORMATIONS POUR LA SEQ ID NO: 133:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 133:
   AGTTCTGCTC CGAAGCTTAG GCAGACTTTT 30
(2) INFORMATIONS POUR LA SEQ ID NO: 135:
   (i) CARACTERISTIQUES DE LA SEQUENCE:
      (A) LONGUEUR: 398 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 135:
   Met Gly Ser Ser His His His His His His Ser Ser Gly Leu Val Pro
   1 5 10 15
   Arg Gly Ser His Met Ala Ser Met Thr Gly Gly Gln Gln Met Gly Arg
   20 25 30
   Ile Leu Glu Arg Ile Leu Glu Asn Trp Asp Gln Cys Asp Thr Gln Thr
   35 40 45
   Leu Arg Lys Lys Arg Phe Ile Phe Phe Cys Ser Thr Ala Trp Pro Gln
   50 55 60
   Tyr Pro Leu Gln Gly Arg Glu Thr Trp Leu Pro Glu Gly Ser Ile Asn
   65 70 75 80
   Tyr Asn Ile Ile Leu Gln Leu Asp Leu Phe Cys Arg Lys Glu Gly Lys
   85 90 95
   Trp Ser Glu Val Pro Tyr Val Gln Thr Phe Phe Ser Leu Arg Asp Asn
   100 105 110
   Ser Gln Leu Cys Lys Lys Cys Gly Leu Cys Pro Thr Gly Ser Pro Gln
   115 120 125
   Ser Pro Pro Pro Tyr Pro Ser Val Pro Ser Pro Thr Pro Ser Ser Thr
   130 135 140
   Asn Lys Asp Pro Pro Leu Thr Gln Thr Val Gln Lys Glu Ile Asp Lys
   145 150 155 160
   Gly Val Asn Asn Glu Pro Lys Ser Ala Asn Ile Pro Arg Leu Cys Pro
   165 170 175
   Leu Gln Ala Val Arg Gly Gly Glu Phe Gly Pro Ala Arg Val Pro Val
   180 185 190
   Pro Phe Ser Leu Ser Asp Leu Lys Gln Ile Lys Ile Asp Leu Gly Lys
   195 200 205
   Phe Ser Asp Asn Pro Asp Gly Tyr Ile Asp Val Leu Gln Gly Leu Gly
   210 215 220
   Gln Ser Phe Asp Leu Thr Trp Arg Asp Ile Met Leu Leu Leu Asn Gln
   225 230 235 240
   Thr Leu Thr Pro Asn Glu Arg Ser Ala Ala Val Thr Ala Ala Arg Glu
   245 250 255
   Phe Gly Asp Leu Trp Tyr Leu Ser Gln Ala Asn Asn Arg Met Thr Thr
   260 265 270
   Glu Glu Arg Thr Thr Pro Thr Gly Gln Gln Ala Val Pro Ser Val Asp
   275 280 285
   Pro His Trp Asp Thr Glu Ser Glu His Gly Asp Trp Cys His Lys His
   290 295 300
   Leu Leu Thr Cys Val Leu Glu Gly Leu Arg Lys Thr Arg Lys Lys Pro
   305 310 315 320
   Met Asn Tyr Ser Met Met Ser Thr Ile Thr Gln Gly Lys Glu Glu Asn
   325 330 335
   Leu Thr Ala Phe Leu Asp Arg Leu Arg Glu Ala Leu Arg Lys His Thr
   340 345 350
   Ser Leu Ser Pro Asp Ser Ile Glu Gly Gln Leu Ile Leu Lys Asp Lys
   355 360 365
   Phe Ile Thr Gln Ser Ala Ala Asp Ile Arg Lys Asn Phe Lys Ser Leu
   370 375 380
   Pro Lys Leu Ala Ala Ala Leu Glu His His His His His His
   385 390 395
(2) INFORMATIONS POUR LA SEQ ID NO: 137:
   (i) CARACTERISTIQUES DE LA SEQUENCE:
      (A) LONGUEUR: 378 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 137:
   Met Ala Ser Met Thr Gly Gly Gln Gln Met Gly Arg Ile Leu Glu Arg
   1 5 10 15
   Ile Leu Glu Asn Trp Asp Gln Cys Asp Thr Gln Thr Leu Arg Lys Lys
   20 25 30
   Arg Phe Ile Phe Phe Cys Ser Thr Ala Trp Pro Gln Tyr Pro Leu Gln
   35 40 45
   Gly Arg Glu Thr Trp Leu Pro Glu Gly Ser Ile Asn Tyr Asn Ile Ile
   50 55 60
   Leu Gln Leu Asp Leu Phe Cys Arg Lys Glu Gly Lys Trp Ser Glu Val
   65 70 75 80
   Pro Tyr Val Gln Thr Phe Phe Ser Leu Arg Asp Asn Ser Gln Leu Cys
   85 90 95
   Lys Lys Cys Gly Leu Cys Pro Thr Gly Ser Pro Gln Ser Pro Pro Pro
   100 105 110
   Tyr Pro Ser Val Pro Ser Pro Thr Pro Ser Ser Thr Asn Lys Asp Pro
           115                 120                 125
   Pro Leu Thr Gln Thr Val Gln Lys Glu Ile Asp Lys Gly Val Asn Asn
       130                 135                 140
   Glu Pro Lys Ser Ala Asn Ile Pro Arg Leu Cys Pro Leu Gln Ala Val
   145                 150                 155                 160
   Arg Gly Gly Glu Phe Gly Pro Ala Arg Val Pro Val Pro Phe Ser Leu
                   165                 170                 175
   Ser Asp Leu Lys Gln Ile Lys Ile Asp Leu Gly Lys Phe Ser Asp Asn
               180                 185                 190
   Pro Asp Gly Tyr Ile Asp Val Leu Gln Gly Leu Gly Gln Ser Phe Asp
           195                 200                 205
   Leu Thr Trp Arg Asp Ile Met Leu Leu Leu Asn Gln Thr Leu Thr Pro
       210                 215                 220
   Asn Glu Arg Ser Ala Ala Val Thr Ala Ala Arg Glu Phe Gly Asp Leu
   225                 230                 235                 240
   Trp Tyr Leu Ser Gln Ala Asn Asn Arg Met Thr Thr Glu Glu Arg Thr
                   245                 250                 255
   Thr Pro Thr Gly Gln Gln Ala Val Pro Ser Val Asp Pro His Trp Asp
               260                 265                 270
   Thr Glu Ser Glu His Gly Asp Trp Cys His Lys His Leu Leu Thr Cys
           275                 280                 285
   Val Leu Glu Gly Leu Arg Lys Thr Arg Lys Lys Pro Met Asn Tyr Ser
       290                 295                 300
   Met Met Ser Thr Ile Thr Gln Gly Lys Glu Glu Asn Leu Thr Ala Phe
   305                 310                 315                 320
   Leu Asp Arg Leu Arg Glu Ala Leu Arg Lys His Thr Ser Leu Ser Pro
                   325                 330                 335
   Asp Ser Ile Glu Gly Gln Leu Ile Leu Lys Asp Lys Phe Ile Thr Gln
               340                 345                 350
   Ser Ala Ala Asp Ile Arg Lys Asn Phe Lys Ser Leu Pro Lys Leu Ala
           355                 360                 365
   Ala Ala Leu Glu His His His His His His
       370                 375
(2) INFORMATIONS POUR LA SEQ ID NO: 138:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 138:
   CTTGGAGGGT GCATAACCAG GGAAT 25
(2) INFORMATIONS POUR LA SEQ ID NO: 139:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 139:
   TGTCCGCTGT GCTCCTGATC 20
(2) INFORMATIONS POUR LA SEQ ID NO: 140:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 140:
   CTATGTCCTT TTGGACTGTT TGGGT 25
(2) INFORMATIONS POUR LA SEQ ID NO: 141:
   (i) CARACTERISTIQUES DE LA SEQUENCE:
      (A) LONGUEUR: 764 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 141:
   TGTCCGCTGT GCTCCTGATC CAGCACAGGC GCCCATTGCC TCTCCCAATT GGGCTAAAGG   60
   CTTGCCATTG TTCCTGCACA GCTAAGTGCC TGGGTTCATC CTAATCGAGC TGAACACTAG  120
   TCACTGGGTT CCACGGTTCT CTTCCATGAC CCATGGCTTC TAATAGAGCT ATAACACTCA  180
   CTGCATGGTC CAAGATTCCA TTCCTTGGAA TCCGTGAGAC CAAGAACCCC AGGTCAGAGA  240
   ACACAAGGCT TGCCACCATG TTGGAAGCAG CCCACCACCA TTTTGGAAGC AGCCCGCCAC  300
   TATCTTGGGA GCTCTGGGAG CAAGGACCCC AGGTAACAAT TTGGTGACCA CGAAGGGACC  360
   TGAATCCGCA ACCATGAAGG GATCTCCAAA GCAATTGGAA ATGTTCCTCC CAAGGCAAAA  420
   ATGCCCCTAA GATGTATTCT GGAGAATTGG GACCAATTTG ACCCTCAGAC AGTAAGAAAA  480
   AAATGACTTA TATTCTTCTG CAGTACCGCC CTGGCCACGA TATCCTCTTC AAGGGGGAGA  540
   AACCTGGCCT CCTGAGGGAA GTATAAATTA TAACACCATC TTACAGCTAG ACCTGTTTTG  600
   TAGAAAAGGA GGCAAATGGA GTGAAGTGCC ATATTTACAA ACTTTCTTTT CATTAAAAGA  660
   CAACTCGCAA TTATGTTAAC AGTGTGATTT GTGTTCCTAC ACGGAAGCCC TCAGATTCTA  720
   CTCCCCACCC CCGGCATCTC CCCTGAATCC CTCCCCAACT TATT                    764
(2) INFORMATIONS POUR LA SEQ ID NO: 142:
   (i) CARACTERISTIQUES DE LA SEQUENCE:
      (A) LONGUEUR: 800 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 142:
   TGTCCGCTGT GCTCCTGATC CAGCACAGGC GCCCATTGCC TCTCCCAATT GGGCTAAAGG   60
   CTTGCCATTG TTCCTGCACA GCTAAGTGCC TGGGTTCATC CTAATCGAGC TGAACACTAG  120
   TCACTGGGTT CCACGGTTCT CTTCCATGAC CCATGGCTTC TAATAGAGCT ATAACACTCA  180
   CTGCATGGTC CAAGATTCCA TTCCTTGGAA TCCGTGAGAC CAAGAACCCC AGGTCAGAGA  240
   ACACAAGGCT TGCCACCATG TTGGAAGCAG CCCACCACCA TTTTGGAAGC GGCCCGCCAC  300
   TATCTTGGGA GCTCTGGGAG CAAGGACCCC CAGGTAACAA TTTGGTGACC ACGAAGGGAC  360
   CTGAATCCGC AACCATGAAG GGATCTCCAA AGCAATTGGA AATGTTCCTC CCAAGGCAAA  420
   AATGCCCCTA AGATGTATTC TGGAGAATTG GGACCAATCT GACCCTCAGA CAGTAAGAAA  480
   AAAAATGACT TATATTCTTC TGCAGTACCG CCTGGCCACG GATATCCTCT TCAAGGGGGA  540
   GAAACCTGGC CTCCTGAGGG AAGTATAAAT TATAACACCA TCTTACAGCT AGACCTGTTT  600
   TGTAGAAAAG GAGGCAAATG GAGTGAAGTG CCATATTTAC AAACTTTCTT TTCATTAAAA  660
   GACAACTCGC AATTATGTAA ACAGTGTGAT TTGTGTCCTA CAGGAAGCCC TCAGATCTAC  720
   CTCCCTACCC CGGCATCTCC CTGACTCCTT CCCCAACTAA TAAGGACCCA CTTCAGCCCA  780
   AACAGTCCAA AAGGACATAG 800